# EUROPEAN PATENT SPECIFICATION

(11) **EP 3 077 370 B1**
(45) Date of publication and mention of the grant of the patent: **04.09.2019**
(21) Application number: 14866821.3
(22) Date of filing: 02.12.2014
(51) Int. Cl.: C07C 265/12, C07C 265/04, C07C 263/10, C07D 317/28, C08G 18/72, C08G 18/73, C08G 18/74, C08G 18/48, C08G 18/58, B32B 27/04, B32B 27/12

(54) **DEGRADABLE ISOCYANATE COMPOUNDS AND APPLICATIONS THEREOF**
ABBAUBARE ISOCYANATVERBINDUNGEN UND ANWENDUNGEN DAVON
COMPOSÉS D'ISOCYANATE DÉGRADABLES ET APPLICATIONS ASSOCIÉES

(30) Priority: 02.12.2013 CN 201310634385
(43) Date of publication of application: 12.10.2016
(73) Proprietor: Adesso Advanced Materials Wuhu Co., Ltd., Wuhu, Anhui 241000 (CN)
(72) Inventor: QIN, Bing, Shanghai 201101 (CN); LI, Xin, Cambridge CB4 0WS (GB); LIANG, Bo, Plainsboro, NJ 08536 (US)
(74) Representative: Rau, Schneck & Hübner Patentanwälte Rechtsanwälte PartGmbB
(86) International application number: PCT/CN2014/001082
(87) International publication number: WO 2015/081610

(56) References cited:
- EP-A1- 0 611 050
- WO-A1-2012/071896
- WO-A1-2013/007128
- WO-A1-2014/169846
- CN-A- 101 151 289
- CN-A- 101 870 686
- CN-A- 103 254 406
- CN-A- 103 694 140
- US-A1- 2013 245 133
- US-A1- 2013 245 133
- DATABASE CA [Online] CHEMICAL ABSTRACTS SERVICE, COLUMBUS, OHIO, US; JANG, DONG GYU: "High refractive index monomer compositions for polyurethanes with good thermal stability for optical lenses", XP002771134, retrieved from STN Database accession no. 2012:848332 -& KR 2012 0060254 A (KOC SOLUTION CO LTD [KR]) 12 June 2012 (2012-06-12)
- Sarah Abramson ET AL: "New Macromolecular Host Systems. Preparation and Structure of Certain Functionalized 2,6-Diaryl-cis-l,3,5,7-Tetraoxadecalin Podand Compounds and Macro-m-cyclophanes", J. CHEM. SOC., CHEM. COMMUN, 1 January 1994 (1994-01-01), XP055381567, Retrieved from the Internet: URL:http://pubs.rsc.org/en/content/article pdf/1994/c3/c39940001611
- N D Ghatue ET AL: "Syntheses of Isocyanates*", Die Angewandte Makromolekulare Chemie, 1 January 1971 (1971-01-01), pages 83-90, XP055381562, Retrieved from the Internet: URL:http://onlinelibrary.wiley.com/store/1 0.1002/apmc.1971.050190107/asset/050190107 _ftp.pdf?v=1&t=j3wx991z&s=2e3ee7bb290208cd 605a5ddd30c6a995c8e7d243
- J A Barth Leipzig ET AL: "Darstellung von lsocyanatderivaten der aliphatischen Diather On the Preparation of Isocyanatoderivatives of Aliphatic Dietliers CH3-CH-0 -[CH1]n-O -CH- CH3 I I NCO I I 3 a-e NH-CO-NH-CjHlo 4 a--P", Band, 1 January 1978 (1978-01-01), page 245, XP055381560, Retrieved from the Internet: URL:http://onlinelibrary.wiley.com/store/1 0.1002/prac.19783200209/asset/19783200209_ ftp.pdf?v=1&t=j3wx60cp&s=d2e25f15fe0b8582c 9f8c95f8f5e0e9e0135cec6
- STEPHANE MENUEL ET AL: "New synthetic approach to per-O-acetyl-isocyanates, isothiocyanates and thioureas in the disaccharide and cyclodextrin series", NEW JOURNAL OF CHEMISTRY, vol. 30, no. 4, 1 January 2006 (2006-01-01), page 603, XP055381538, GB ISSN: 1144-0546, DOI: 10.1039/b600023a
- DRAGOS GHERASE ET AL: "XRD and VCD: a marriage of love or convenience? Honeymoon around a cyclic urea derivative", ACTA CRYSTALLOGRAPHICA SECTION C. CRYSTAL STRUCTURE COMMUNICATIONS, vol. 68, no. 7, 13 June 2012 (2012-06-13), pages o247-o252, XP055381820, DK ISSN: 0108-2701, DOI: 10.1107/S010827011202358X

## Description

### Related Application

This application claims priority to Chinese Application Number 201310634385.2, filed on December 2, 2013.

### Background of the Invention

Due to the unique structure and excellent property, polyurethane material of the polyfoam, elastomer and adhesive are widely used in the construction, automobile industry, national defense and aerospace etc.

The backbone of polyurethane contains the high molecular compound with a repeating segment of polyurethane. The polyurethane belongs to the synthetic material which has its wide-range in application. Up to now, the polyurethane with its widely use in the biomedical polymer material is obtained through the process that the prepolymer of the isocyanate terminated matrix, synthesized by the macromolecule dihydric alcohol, and the excess of diisocyanate can combine with low molecular dibasic alcohol or diamine to carry the chain reaction. Thereinto, the macromolecule polyhydric alcohols becomes the flexible chain; diisocyanate and chain-extender turn into the rigid chain. Take the frequently-used diisocyanate and dihydric alcohol as the examples and the reaction formula is as follows:

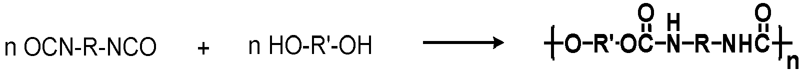

The flexible chain of polyurethane is generally constituted by the polyether or polyester with weak polarity, which reflects its elastic properties. The glass transition temperature, tensile strength, elongation, abrasion resistance, shear strength, blood compatibility and the hydrophilic properties of polyurethane can be regulated by the molecular design and choose different types of flexible chain or different molecular weights of rigid chain, or combine several kinds of flexible chain and rigid chain into the application to make the polyurethane possess the specific properties. The polymerization could generate the block or cross-linked polymer. More than the carbamate included in the polyurethane macromolecule, the ether, ester, urea, biuret, allophanate matrix etc could be also contained. The structures of polyurethane macromolecule are changeful, whose properties could be adjusted over a wide range. Different numbers and different types of functional groups take different synthesis crafts to prepare the polyurethane products with different varieties and properties.

Under natural conditions, the vast majority of plastics and other macromolecule materials currently used are non-degradable and the heavy use of macromolecule materials could cause the serious problems of white pollution, and then the environment was destroyed enormously. With the gradually deteriorating environmental problems of global warming, pollution arised in the earth, the environmental protection is urgently required to take actions to deal with. Now, many countries have made the legislation to restrict the use of non-degradable single-used plastic bags and promote the use of disposable shopping bags, garbage bags made by biodegradable plastic. In the packaging or other industry, polyurethane foam plastics, which possess excellent high specific strength, good insulation properties, fine vibration cushioning properties and other characteristics, can be used as the high-grade packaging materials. Polyurethane material with good biocompatibility anti-thrombotic property has the advantages of excellent mechanical properties, easy processing, low price, etc. So it has a broad application prospects in the biomedical field. But these hardly degradable polyurethane plastics have brought the environmental pollution problems for the industrial development. Therefore, the degradation property of polyurethane material has a crucial importance in the application of packaging and medical industry. For the single-used packaging material, the degradable polyurethane foaming plastics could reduce the environmental pollution to protect the environment. Moreover, the degradable medical polyurethane could be biodegraded and absorbed by human body. And in the meantime, it can be used as the excellent tissue engineering, alternative materials for drug delivery to promote the technical progress of medicine greatly.

Currently, the polyurethane recycling methods contain the physical recycling, incineration recycling and chemical recycling. The physical recycling methods do not destroy the chemical structure of the eupolymer and have not changed its composition. The polyurethane could be reused as the filler, compression molding and other purposes. The physical recycling method of polyurethane is simple to execute. However, the market of the products obtained by this method is limited and the technical limitations of process also arised. The recycling waste is mainly the low-grade scrap recycling polyurethane waste. And the incineration recycling method mainly takes the incineration method to obtain energy from the polyurethane waste, whilst a large amount of toxic would be discharged to cause serious environmental pollution on the condition that the incomplete combustion of polyurethane happens in the incineration process. The purpose of chemical recycling method is to degrade the polyurethane into the reused the liquid oligomer or the organic compound with small molecule under the condition of the chemical reagents and catalysts applied in the polyurethane. The recycling of raw material would be achieved by above-mentioned chemical method. However, due to its limitations of price and cost, the technical skills of degradable polyurethane are still immature with the low marketization and commercialization. So the fundamental research of the degradable polyurethane technology should be carried out systematically and thoroughly.

Furthermore, compared with the traditional epoxy resin, the polyurethane modified epoxy resin has excellent properties in its toughness and shock strength. Because of the fine mechanical properties, electrical insulation, bonding properties, the polyurethane is widely used in the composite, casting parts, electronic appliances, paint and other industry. Of the fiber-reinforced epoxy composites, the carbon fiber composite has been put into the application of aviation, automobiles, trains, ships, wind power, tidal energy, sporting goods and other industries. By 2015, the global production of composite materials will be increase greatly of over 10 million tons. However, the high density and three-dimensional network structure of cured epoxy resin would make itself become the extremely hard and durable materials, which can withstand the effects of a wide range of environmental conditions. Meanwhile, the cross-linked network structure of cured epoxy resin could hardly make itself removed, recycled to reuse. Essentially, the cross-linked reaction through the process of polyamine and epoxy resin compound is irreversible. Thus this substance can't be remelted, reformed without damage and easily dissolved. And the dealing and recycling of fiber composite waste would be the worldwide problems to constrain the sustainable development of fiber composites.

Up to now, the recovery process of fiber composite has the followed methods: 1. High temperature thermal degradation (Thermochimica Acta 2007(454):109-115), by which clean filler and fibre can be recycled, but needs to be done under the condition of high temperature, requiring high standard of equipment; 2. fluidized bed (Applied surface science 2008 (254): 2588-2593), also needs to be done under the condition of high temperature to recycle the clean fibre; 3. supercutical fluid (water (Materials and design 2010 (31): 999-1002), alcohol (Ind.eng.chem.res. 2010 (49): 4535-4541), carbon dioxide (CN102181071),etc) also achieves the degradation of epoxy resin system, but still in labortray stage and has a long way from the real industrialization; 4. Applying nitric acid (Journal of applied polymer science, 2004 (95): 1912-1916) to degrade the epoxy resin, the recycled fibre is clean, however, strong acid like nitric acid is highly corrosive, requires high standard of equipment, and operation security is low but the recycling cost is high, the post-processing is also difficult to realize. In general, these methods have limitations of different levels, such as fiber shortening, performance degradation, environmental pollution and high cost of recycling. Thus, the effective degradation method for recycling the composite waste is also the urgent problem to be solved in the composite industry.

Diisocyanates containing ether linkages are already known from e.g. EP 0 611 050 A1.

### Summary of the Invention

Aiming at the problems of the existing technology, this applicant provides a method for degradable isocyanate and its preparation ways of the polyurethane modified epoxy resin synthesized by the isocyanate and epoxy resin, the polyurethane and reinforced composites synthesized by the isocyanate and dyhydroxy or polyol compound, the polyurethane modified polymer, the polyurethane polymer and reinforced composite. The degradable composites prepared by using this invention have good mechanical property, and are widely used in the application industry of different composites. Under the specific conditions, the composite could be degraded. And the degradable products of reinforced material and polymer matrix could be also separated and recycled. Moreover, the degradation recovery method of composite could be easily and economically controlled under the mild reaction conditions.

In one aspect, the present invention provides isocyanate compounds having at least two isocyanate groups, wherein each of the isocyanate groups is bonded via a linker to a linear or cyclic oxy-carbohydro moiety that contains oxygen atoms not less than the isocyanate groups and optionally contains one or more heteroatoms each independently being S or N; each of the linker is alkylene, heteroalkylene, alkenylene, heteroalkyl, alkynylene, heteroalkynlene, arylene, or heteroaryl; the linear oxy-carbohydro moiety is alkyl, alkenyl, or alkynyl optionally substituted in the main chain with one or more heteroatoms, and is connected via oxo to each of the linkers that link the isocyanate groups to the oxy-carbohydro moiety; and the cyclic oxy-carbohydro is saturated, unsaturated, or aromatic ring or fused ring containing at least one oxygen atom in the ring and optionally contain an oxygen in a ring substituent that is bonded to the linker with an isocyanate group. As used herein, the term oxy-carbohydro refers to a functional group which has a carbohydro core or moiety that has two or more oxygen atoms each connecting the carbohydro moiety with the linker to an isocyanate group. For illustration, an example of such oxy-carbohydro is CH₃CH(-O-)₂ which contains two oxo groups and -CH(CH₃)- as the carbohydro moiety.

In some embodiments, the present invention provides isocyanate compounds of Formula (I) shown below: 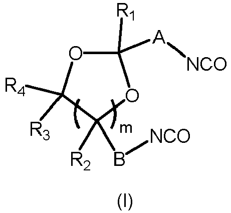 In Formula (I),
m is 1, 2, 3, 4, or 5;
each of R₁, R₂, R₃ and R₄ independently is hydrogen, alkyl, cycloalkyl, heterocyclic, heterocyclic, alkenyl, cycloalkenyl, alkynyl, aryl, heteroaryl, alkylene-oxy-alkyl, alkylene-oxy-alkyl, alkylene-oxy-hetero-cyclic, alkylene-oxy-hetero-cycloalkyl, alkylene-oxy-alkenyl, alkylene-oxy-cycloalkenyl, alkylene-aryl, alkylene-oxy-heteroaryl, cycloalkylene-oxy-alkyl, cycloalkylene-oxy-cycloalkyl, cycloalkylene-oxy-heterocyclic, cycloalkylene-oxy-heterocycloalkyl, cycloalkylene-oxy-alkenyl, cycloalkylene-oxy-cycloalkenyl, cycloalkylene-oxy-aryl, cycloalkylene-oxy-heteroaryl, heterocycloalkylene-oxy-alkyl, heterocycloalkylene-oxy-cycloalkyl, heterocycloalkylene-oxy-heterocyclic, heterocycloalkylene-oxy-heterocycloalkyl, heterocycloalkylene-oxy-alkenyl, heterocycloalkylene-oxy-cycloalkenyl, heterocycloalkylene-oxy-aryl, heterocycloalkylene-oxy-heteroaryl, arylene-oxy-alkyl, arylene-oxy-cycloalkyl, arylene-oxy-heterocyclic, arylene-oxy-heterocycloalkyl, arylene-oxy-alkenyl, arylene-oxy-cycloalkenyl, arylene-oxy-aryl, or arylene-oxy-heteroaryl; or
R₃ and R₄, together with the carbon atom to which they are bonded, form a 3-7 membered ring optionally containing one or more heteroatoms each of which is independently S, O, or N; or
R₁ and A; together with the carbon atom to which they are bonded, form a 3-7 membered ring optionally containing one or more heteroatoms each of which is independently S, O, or N; or
R₂ and B, together with the carbon atom to which they are bonded, form a 3-7 membered ring optionally containing one or more heteroatoms each of which is independently S, O, or N; or
each of A and B independently is alkylene, alkylene-hetero-alkylene, alkenylene, alkenylene-hetero-alkenylene, alkylene-hetero-alkenylene, alkynylene, cycloalkylene, alkylene-cycloalkylene, alkylene-cycloalkylene-alkylene, alkenylene-cycloalkylene, alkenylene-cycloalkylene-alkenylene, alkylene-cycloalkylene-alkenylene, alkynylene-cycloalkylene, alkynylene-cycloalkylene-alkynylene, heterocycloalkylene, alkylene-heterocycloalkylene, alkylene-heterocycloalkylene-alkylene, alkenylene-heterocycloalkylene, alkenylene-heterocycloalkylene-alkenylene, alkylene-heterocycloalkylene-alkenylene, alkynylene-heterocycloalkylene, alkynylene-heterocycloalkylene-alkynylene, cycloalkenylene, alkylene-cycloalkenylene, alkylene-cycloalkenylene-alkylene, alkenylene-cycloalkenylene, alkenylene-cycloalkenylene-alkenylene, alkylene-cycloalkenylene-alkenylene, alkynylene-cycloalkenylene, alkynylene-cycloalkenylene-alkynylene, heterocycloalkenylene, alkylene-heterocycloalkenylene, alkylene-heterocycloalkenylene-alkylene, alkenylene-heterocycloalkenylene, alkenylene-heterocycloalkenylene-alkenylene, alkylene-heterocycloalkenylene-alkenylene, alkynylene-heterocycloalkenylene, alkynylene-heterocycloalkenylene-alkynylene, Arylene, alkylene-arylene, alkylene-arylene-alkylene, alkenylene-arylene, alkenylene-arylene-alkenylene, alkylene-arylene-alkenylene, alkynylene-arylene, alkynylene-arylene-alkynylene, Heteroarylene, alkylene-heteroarylene, alkylene-heteroarylene-alkylene, alkenylene-heteroarylene, alkenylene-heteroarylene-alkenylene, alkylene-heteroarylene-alkenylene, alkynylene-heteroarylene, alkynylene-heteroarylene-alkynylene, carbonyl, or thiocarbonyl.

In some narrower embodiments, m is 1; each of R₁, R₂, R₃ and R₄ is independently hydrogen or alkyl; or each A and B independently is alkylene or alkenylene.

Isocyanate compounds of Formula (II) are disclosed: 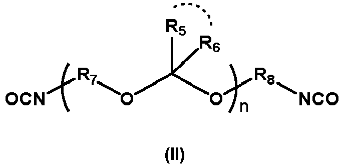 In Formula (II),
each of R₅ and R₆ independently is hydrogen, alkyl, cylcoalkyl, heterocyclic, heterocycloalkyl, alkenyl, cycloalkenyl, aryl, heteroaryl, alkyl-hetero-alkyl, alkynyl, alkylene, alkylene-hetero-alkylene, alkenylene, alkylene-hetero-alkenylene, alkynylene, or alkylene-hetero-alkynylene; or, R₅ and R₆, together with the carbon atom to which they are bonded, form a 3-7 membered ring optionally containing one or more heteroatoms each of which is independently S, O, or N;
n is 1, 2, 3, 4, 5, or 6;
each of R₇ and R₈ is independently alkylene, alkylene-hetero-alkylene, alkenylene, alkenylene-hetero-alkenylene, alkylene-hetero-alkenylene, alkynylene, cycloalkylene, alkylene-cycloalkylene, alkylene-cycloalkylene-alkylene, alkenylene-cycloalkylene, alkenylene-cycloalkylene-alkenylene, alkylene-cycloalkylene-alkenylene, alkynylene-cycloalkylene, alkynylene-cycloalkylene-alkynylene, heterocycloalkylene, alkylene-heterocycloalkylene, alkylene-heterocycloalkylene-alkylene, alkenylene-heterocycloalkylene, alkenylene-heterocycloalkylene-alkenylene, alkylene-heterocycloalkylene-alkenylene, alkynylene-heterocycloalkylene, alkynylene-heterocycloalkylene-alkynylene, cycloalkenylene, alkylene-cycloalkenylene, alkylene-cycloalkenylene-alkylene, alkenylene-cycloalkenylene, alkenylene-cycloalkenylene-alkenylene, alkylene-cycloalkenylene-alkenylene, alkynylene-cycloalkenylene, alkynylene-cycloalkenylene-alkynylene, heterocycloalkenylene, alkylene-heterocycloalkenylene, alkylene-heterocycloalkenylene-alkylene, alkenylene-heterocycloalkenylene, alkenylene-heterocycloalkenylene-alkenylene, alkylene-heterocycloalkenylene-alkenylene, alkynylene-heterocycloalkenylene, alkynylene-heterocycloalkenylene-alkynylene, Arylene, alkylene-arylene, alkylene-arylene-alkylene, alkenylene-arylene, alkenylene-arylene-alkenylene, alkylene-arylene-alkenylene, alkynylene-arylene, alkynylene-arylene-alkynylene, Heteroarylene, alkylene-heteroarylene, alkylene-heteroarylene-alkylene, alkenylene-heteroarylene, alkenylene-heteroarylene-alkenylene, alkylene-heteroarylene-alkenylene, alkynylene-heteroarylene, alkynylene-heteroarylene-alkynylene, 1,4-alkyl substituted piperazine, carbonyl, or thiocarbonyl.

In some other disclosures, each of R₅ and R₆ independently is hydrogen or alkyl; or, R₅ and R₆, together with the carbon atom to which they are bonded, form a 3-6 membered ring optionally containing one or more heteroatoms each of which is independently S, O, or N; n is 1; or each of R₇ and R₈ is independently alkylene, alkylene-hetero-alkylene, or alkenylene.

An isocyante compound of Forumula (III) is also disclosed:

(R₉)ₚR(-O-R₁₀-N=C=O)_{q} (III)

In Formula (III),
R and the oxygen atoms (each between R and R₁₀) together constitute the the linear or cyclic oxy-carbohydro moiety that optionally contains one or more heteroatoms each independently being S or N;
p is an integer no less than 0;
q is an integer of at least 3; when R is a carbon atom, the sum of p and q is 4;
each R₉ independently is hydrogen, alkyl, cylcoalkyl, heterocyclic, heterocycloalkyl, alkenyl, cycloalkenyl, aryl, heteroaryl, alkyl-hetero-alkyl, alkynyl, alkylene, alkylene-hetero-alkylene, alkenylene, alkylene-hetero-alkenylene, alkynylene, or alkylene-hetero-alkynylene; or, two R₉, together with the carbon atom to which they are bonded, form a 3-7 membered ring optionally containing one or more heteroatoms each of which is independently S, O, or N; and
each R₁₀ independently is alkylene, alkylene-hetero-alkylene, alkenylene, alkenylene-hetero-alkenylene, alkylene-hetero-alkenylene, alkynylene, cycloalkylene, alkylene-cycloalkylene, alkylene-cycloalkylene-alkylene, alkenylene-cycloalkylene, alkenylene-cycloalkylene-alkenylene, alkylene-cycloalkylene-alkenylene, alkynylene-cycloalkylene, alkynylene-cycloalkylene-alkynylene, heterocycloalkylene, alkylene-heterocycloalkylene, alkylene-heterocycloalkylene-alkylene, alkenylene-heterocycloalkylene, alkenylene-heterocycloalkylene-alkenylene, alkylene-heterocycloalkylene-alkenylene, alkynylene-heterocycloalkylene, alkynylene-heterocycloalkylene-alkynylene, cycloalkenylene, alkylene-cycloalkenylene, alkylene-cycloalkenylene-alkylene, alkenylene-cycloalkenylene, alkenylene-cycloalkenylene-alkenylene, alkylene-cycloalkenylene-alkenylene, alkynylene-cycloalkenylene, alkynylene-cycloalkenylene-alkynylene, heterocycloalkenylene, alkylene-heterocycloalkenylene, alkylene-heterocycloalkenylene-alkylene, alkenylene-heterocycloalkenylene, alkenylene-heterocycloalkenylene-alkenylene, alkylene-heterocycloalkenylene-alkenylene, alkynylene-heterocycloalkenylene, alkynylene-heterocycloalkenylene-alkynylene, Arylene, alkylene-arylene, alkylene-arylene-alkylene, alkenylene-arylene, alkenylene-arylene-alkenylene, alkylene-arylene-alkenylene, alkynylene-arylene, alkynylene-arylene-alkynylene, Heteroarylene, alkylene-heteroarylene, alkylene-heteroarylene-alkylene, alkenylene-heteroarylene, alkenylene-heteroarylene-alkenylene, alkylene-heteroarylene-alkenylene, alkynylene-heteroarylene, alkynylene-heteroarylene-alkynylene, 1,4-alkyl substituted piperazine, carbonyl, or thiocarbonyl.

Examples of the isocyanate compounds provided by the present invention include 2,4-bis(isocyanatomethyl)-1,3-dioxolane.

Examples of the isocyanate compounds disclosed herein include bis(4-isocyanatophenoxy)methane, bis(2-isocyanatoethoxy)methane, 1,1,1-tris(2-isocyanatoethoxy)ethane, 1,1,2-tris(2-isocyanatoethoxy)ethane, tetrakis(2-isocyanatoethoxy)methane, 1,1,1,2-tetrakis(2-isocyanatoethoxy)ethane, and 4,4',4"-(ethane-1,1,1-triyltris(oxy))tris(isocyanatobenzene).

In still another aspect, the present invention provides a method for preparing an isocyanate compound of the invention as described above. The method includes at least the step of converting a compound of Formula (I-A)to the isocynate compound, wherein R, R₁, R₂, R₃, R₄, A, B, R₅, R₆, R₇, R₈, R₉, R₁₀, m, n, p, and q, when present, are the same as those set forth above for the isocyanate compounds of this invention.

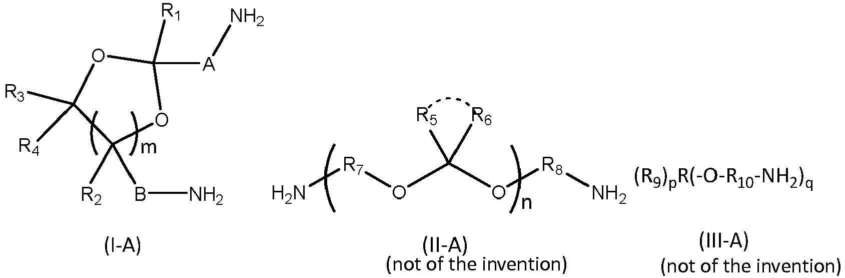

In some embodiments of this method, the conversion of the compound of Formula (I-A) to the desired isocyanate compound is by reacting the compound of Formula (I-A) with phosgene, triphosgene, or trichloromethyl chloroformate, optionally at the presence of a catalyst. In this reaction, e.g., the molar ratio of the compound of Formula (I-A) to phosgene, triphosgene, or trichloromethyl chloroformate is 1:2∼100, the reaction temperature is in the range of -20∼150°C; and the catalyst, when present, comprises an amine, a pyridine derivative, or N,N dimethyl formamide.

In yet still another aspect, the present invention provides another method for preparing an isocyanate compound of the invention described above. The method includes the step of converting a compound of Formula (I-B) to the isocyanate compound, wherein R, R₁, R₂, R₃, R₄, A, B, R₅, R₆, R₇, R₈, R₉ R₁₀, m, n, p, and q, when present, are the same as those in the isocyanate compound, and each X is independently hydroxyl, thiol, or trimethylsiloxy. 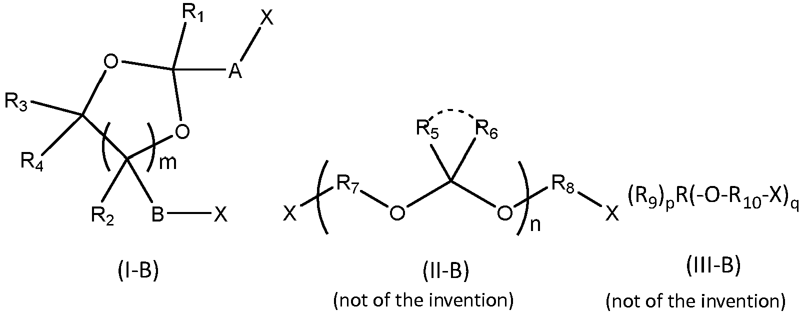 In this method, e.g., the conversion of the compound of Formula (I-B) to the desired isocyanate compound can be by reacting the compound of Formula (I-B) with tetrabutylammonium cyanate, optionally with the presence of a catalyst. In such a reaction, the molar ratio of the compound of Formula (I-B) to tetrabutylammonium cyanate can be in the range of 1:2∼100, the reaction temperature can be in the range of -20∼150 °C; and the catalyst, when present, includes a triazine compound.

In still another aspect, the present invention provides degradable polyurethanes, wherein each of the polyurethanes is made by polymerizing an isocyanate compound of the invention as described above with a hydrogen-donating compound which comprises a dihydric alcohol, a polyhydric alcohol, polyetherpolyol, polyesterpolyol, binary mercaptan, polybasic mercaptan, phenol, carboxylic acid, urea, amide, diamine, or polyamine; and the polyurethane has a cleavable cross-linking structure of Formula (I-C), wherein R, R₁, R₂, R₃, R₄, A, B, R₅, R₆, R₇, R₈, R₉, R₁₀, m, n, p, and q, when present, are the same as those in the isocyanate compounds of the invention described above. 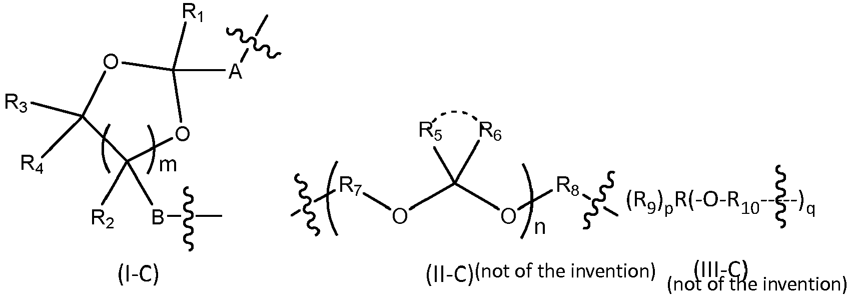 Similarly, the present invention also provides degradable cross-linked polymers each of which is made by polymerizing an isocyanate compound of the invention as described above with an epoxy resin and a degradable curing agent. The epoxy resin used can include a glycidyl ether epoxy resin, a glycidyl ester epoxy resin, glycidyl epoxy amine epoxy resin, a trifunctional epoxy resin, a tetrafunctional epoxy resin, a novolac epoxy resin, an o-cresol formaldehyde epoxy resin, an aliphatic epoxy resin, an alicyclic epoxy resin, or a nitrogen-containing epoxy resin; and the degradable curing agent can include an acetal or ketal aliphatic amine (as described in, e.g., WO 2012/071896, WO 2013/007128, and CN 103249712A), an acetal or ketal aromatic amine or salt thereof (as described in, e.g., CN 103254406A), an acetal or ketal polyamine (as described in, e.g., CN 103012747A), a cyclic acetal or ketal amine (as described in, e.g., CN 103242509A), an acetal or ketal hydrazide (as described in, e.g., CN 103193959A), or hydrazine (as described in, e.g., CN 201310440092.0); and the cross-linked polymer has a cleavable cross-linking structure of Formula (I-C), wherein R, R₁, R₂, R₃, R₄, A, B, R₅, R₆, R₇, R₈, m, and n, when present, are the same as those in the isocyanate compound.

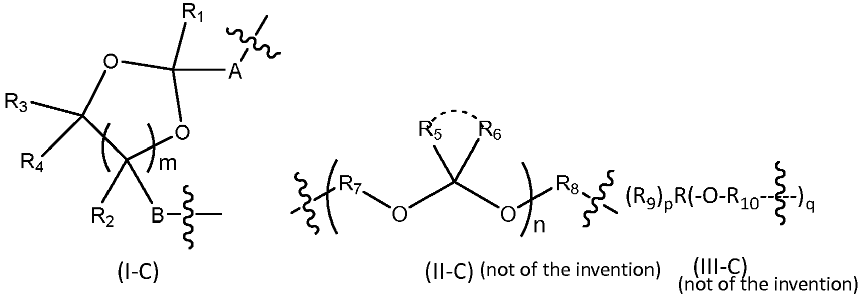

The present invention in yet another aspect further provides a method for degrading a polyurethane or a cross-linked polymer of this invention as just described above. Such a method includes at least the following steps: (1) under the heating and stirring conditions, the degradable polyurethane or the degradable cross-linked polymer is immersed in a mixed acid and solvent system for the degradation for 1^{∼}600 hours at a temperature within the range of 15^{∼}400°C, wherein the mass concentration of acid in the solvent 0.1^{∼}99%; and (2) using an alkali solution to adjust the pH of the degradation solution to above 6 at a temperature within the range of 0^{∼}200°C, wherein the mass concentration of alkali solution is 0.1^{∼}99%.

In some embodiments of this method, the acid comprises hydrochloric acid, hydrobromic acid, hydrofluoric acid, acetic acid, trifluoroacetic acid, lactic acid, formic acid, propionic acid, citric acid, methanesulfonic acid, p-toluenesulfonic acid, nitric acid, sulfuric acid, sulfurous acid, phosphoric acid, perchloric acid, benzoic acid, salicylic acid, or phthalic acid; the solvent system comprises methanol, ethanol, ethylene glycol, propanol, isopropanol, butanol, isobutanol, t-butanol, pentanol, hexanol, heptanol, octanol, nonanol, benzyl alcohol, phenethyl alcohol, p-hydroxymethyl benzene, m-hydroxymethyl benzene, o-hydroxy benzene, p-hydroxyethyl benzene, m-hydroxyethyl benzene, o-hydroxyethyl benzene, water, N,N-dimethylformamide, N,N-dimethylacetamide, N-methylpyrrolidone, dimethyl sulfoxide, tetrahydrofuran, methyl tetrahydrofuran, glycerol, or dioxane; the alkali comprises lithium hydroxide, sodium hydroxide, potassium hydroxide, calcium hydroxide, sodium carbonate, sodium bicarbonate, potassium carbonate, potassium bicarbonate, or ammonia; the solvent of the alkali solution comprises methanol, ethanol, ethylene glycol, propanol, isopropanol, butanol, isobutanol, t-butanol, pentanol, hexanol, heptanol, octanol, nonanol, benzyl alcohol, phenethyl alcohol, p-hydroxymethyl benzene, m-hydroxymethyl benzene, o-hydroxy benzene, p-hydroxyethyl benzene, m-hydroxyethyl benzene, o-hydroxyethyl benzene, water, N,N-dimethylformamide, N,N-dimethylacetamide, N-methylpyrrolidone, dimethyl sulfoxide, tetrahydrofuran, methyl tetrahydrofuran, glycerol, or dioxane.

In some other embodiments of this method, in step (1), the heating temperature is within the range of 80^{∼}150°C, the heating time is within the range of 4^{∼}8 hours, and the mass concentration of acid in the solvent 0.5^{∼}20%; in step (2), the temperature is within the range of 5^{∼}50 °C, the final pH value after adjustment with the alkali solution is in the range of 6^{∼}12, and the mass concentration of alkali solution is within the range of 5^{∼}30%.

Also provided by the present invention is a recyclable reinforced composite material which includes a polyurethane or a cross-linked polymer as just described above, a reinforcing material, and an auxiliary material. The reinforcing material can include, e.g., carbon nanotubes, boron nitride nanotubes, carbon black, metal nano-particles, metal oxide nanoparticles, organic nanoparticles, iron oxide, glass fibers, carbon fibers, natural fibers, synthetic fibers and fabric made therefrom; and the auxiliary material include, among others, an accelerator, a diluent, a plasticizer, a toughening agent, a thickening agent, a coupling agent, a defoamer, a flatting agent, an ultraviolet absorber, an antioxidant, a brightener, a fluorescent agent, a pigment, or a filler.

Also within the scope of the present invention is a method for recycling a reinforced composite material comprising the steps of: (1) under the heating and stirring conditions, immersing the reinforced composite material in a solution comprising an acid and a solvent and then heating the mixture at a temperature within the range of 15^{∼}400°C for 1^{∼}600 hours to give rise to a degradation solution, wherein the mass concentration of acid in the solution is 0.1^{∼}99%; (2) using an alkali solution of 0^{∼}200°C to adjust the pH value of the degradation solution from step (1) to be greater than 6 to obtain a precipitate, wherein the mass concentration of the alkali in the alkali solution is 0.1^{∼}99%; and (3) separate, wash and dry the precipitate obtained in step (2).

In some embodiments, the acid comprises hydrochloric acid, hydrobromic acid, hydrofluoric acid, acetic acid, trifluoroacetic acid, lactic acid, formic acid, propionic acid, citric acid, methanesulfonic acid, p-toluenesulfonic acid, nitric acid, sulfuric acid, sulfurous acid, phosphoric acid, perchloric acid, benzoic acid, salicylic acid, or phthalic acid; the solvent comprises at least one of methanol, ethanol, ethylene glycol, propanol, isopropanol, butanol, isobutanol, t-butanol, pentanol, hexanol, heptanol, octanol, nonanol, benzyl alcohol, phenethyl alcohol, p-hydroxymethyl benzene, m-hydroxymethyl benzene, o-hydroxy benzene, p-hydroxyethyl benzene, m-hydroxyethyl benzene, o-hydroxyethyl benzene, water, N,N-dimethylformamide, N,N-dimethylacetamide, N-methylpyrrolidone, dimethyl sulfoxide, tetrahydrofuran, methyl tetrahydrofuran, glycerol, or dioxane; the alkali comprises lithium hydroxide, sodium hydroxide, potassium hydroxide, calcium hydroxide, sodium carbonate, sodium bicarbonate, potassium carbonate, potassium bicarbonate, or ammonium hydroxide; and the alkali solvent comprises methanol, ethanol, ethylene glycol, propanol, isopropanol, butanol, isobutanol, t-butanol, pentanol, hexanol, heptanol, octanol, nonanol, benzyl alcohol, phenethyl alcohol, p-hydroxymethyl benzene, m-hydroxymethyl benzene, o-hydroxy benzene, p-hydroxyethyl benzene, m-hydroxyethyl benzene, o-hydroxyethyl benzene, water, N,N-dimethylformamide, N,N-dimethylacetamide, N-methylpyrrolidone, dimethyl sulfoxide, tetrahydrofuran, methyl tetrahydrofuran, glycerol, or dioxane.

In still some other embodiments, in step (1), the mass concentration of acid in the solvent is within the range of 0.5^{∼}20%, the temperature is within the range of 80^{∼}200°C, and the reaction time is 2^{∼}12 hours; and in step (2), the mass concentration of alkali solution is within the range of 5^{∼}30%, the temperature is within the range of 5^{∼}60°C.

A degradable isocyanate provided by the present invention and a macrodihydric alcohol can be used to prepare a degradable polyurethane matrix of this invention. Under the action of an acid, the cleavage or breaking of a particular chemical bond in the polyurethane results in the degradation of the polymer matrix. This degradation process may be performed under relatively mild, economical, and easily controlled reaction conditions. Therefore, the degradable polyurethanes of the present invention have significant environmental and economic advantages over conventional polyurethanes.

Due to their unique structure and excellent performance, polyurethane materials of polyfoam, elastomers, adhesives and others are widely used in construction, automotive, defense, aerospace and other fields. Currently, research of degradable polyurethanes is mostly focused on linear polyurethanes. However, these linear polyurethanes have poor mechanical properties and cannot undergo complete degradation. Degradable cross-linked polyurethanes of the present invention unexpectedly have much better mechanical properties and more complete degradation capability than linear polymers with a similar structure.

The degradable polyurethanes of this invention or polyurethane-modified epoxy resin can combine with degradable glass fibers, carbon fibers, natural fibers, synthetic fibers, or other fiber composite material to obtain the composite materials under the standard or common procedures of preparing composites materials. The composite materials can also be prepared by the combination of degradable polyurethane or polyurethane-modified epoxy resin with non-fibrous materials such as carbon nanotubes, boron nitride nanotubes, carbon black, metal nanoparticles, metal oxide nanoparticles, organic nanoparticles, iron oxide, or other non-fibrous materials.

A degradable composite material is typically degraded in the following manner: After a composite material is immersed in a hot recovery solution of acid and solvent, the polymer matrix would decompose first and then the reinforcing material can be separated and the polymer matrix can be recovered, e.g., after neutralizing the degradation solution with an alkaline solution to produce a precipitate. Under such conditions, the polymer matrix can be decomposed because it is an acid-sensitive cross-linked structure in which the bond cleavage of the acid-sensitive groups will occur. That will cause the crosslinked structure of the polymer matrix to be dissolved in a non-crosslinked polymer (e.g. a thermoplastic polymer) of an organic decomposition solvent. When the non-crosslinked polymer is fully dissolved, the reinforcing materials (e.g., carbon fibers) can be separated and removed from the degradation solution. The degradable polymer matrix yield can be recovered through the process of neutralization, sedimentation and solid-liquid separation. The reinforcing materials and recycled non-crosslinked polymers can therefore be separated, recovered and reused.

As discussed above, the present invention provides degradable polyurethanes and degradable cross-linked polymers - both of which are based on the degradable isocyanate compounds of this invention - and composite materials based on these degradable polyurethanes. Additionally, the present invention provides composite materials that can be prepared from degradable polyurethane-modified epoxy resin, curing agent, auxiliary materials and reinforcing materials (e.g., carbon fibers, glass fibers, synthetic fibers and natural fibers, etc.). Alternatively, the composite materials of this invention can be prepared from degradable polyurethane, auxiliary material and reinforced material. These composite materials can be degraded under relatively mild conditions, and 95% of reinforcing materials (e.g. carbon fibers, glass fibers, synthetic fibers and natural fibers, etc.) can be recovered to retain their original texture and the mechanical properties mostly, and they could be also reused, e.g., to prepare new composite materials. The recovered polymer matrix degradation products can be processed for the usage of plastic products. Such advantageous and efficient utilities of the degradable polyurethanes, degradable cross-linked polymers, and the composite materials made therefrom have never been reported, and the excellent efficiency, ease of handling and economic features of the recovery process also unexpectedly shown advantages over the conventional technologies and products.

The present invention illustrates that during the degradation process of polyurethane or polyurethane-modified epoxy resin composite material provided by the present invention, the cross-linked structure of polyurethane polymer matrix or polyurethane-modified epoxy resin composite material could be broken due to cleavage of specific chemical bonds, which leads to the degradation of the polymer matrix. And the cross-linked structure could be transformed into a non-crosslinked polymer (e.g. a thermoplastic polymer) that could be dissolved in an organic solvent. When the non-crosslinked polymer is fully dissolved in an organic solvent, the reinforced materials can be removed from the solution thereby recovered for potential reuse. The degradation product of the polymer matrix can be recovered through the process of neutralization, sedimentation and solid-liquid separation. The reinforcing materials and recycled non-crosslinked polymers can also be separated, recovered and reused.

The currently composites recovery technology requires incinerating the plastic component of a composite to recover the reinforcing material. However, as a significant advantage, the plastic component of degradable composite materials of this invention and the reinforcing materials used to make the composite materials can both be recycled more efficiently. For instance, the cross-linked polymers of this invention could be degraded to form a thermoplastic polymer wherein, after a small amount of acetal matrix has been lost, the mass recovery ratio of the thermoplastic polymer is high and this polymer can be processed for the industrial use. Also, the mass recovery ratio of the cross-linked polymer of this invention and reinforcing material used therein is greater than 96%. Under the acid recycling conditions, the recycled reinforcement has the stable property with the clean surface and no defects. The recovery methods of the degradable composites of this invention can be carried out under mild conditions, and are economic and easy to control.

As used herein, the term "alkyl," when used alone or as part of a larger moiety (e.g., as in "alkyl-hetero-*alky*/"), refers to a saturated aliphatic hydrocarbon group. It can contain 1 to 12 (e.g., 1 to 8, 1 to 6, or 1 to 4) carbon atoms. As a moiety, it can be denoted as -CₙH₂ₙ₊₁. An alkyl group can be straight or branched. Examples of alkyl groups include, but are not limited to, methyl, ethyl, propyl, isopropyl, butyl, isobutyl, sec-butyl, tert-butyl, n-pentyl, n- heptyl, and 2-ethylhexyl. An alkyl group can be substituted with one or more substituents. When an alkyl is preceded by a carbon-number modifier, e.g., C₁₋₈, its means the alkyl group contains 1 to 8 carbon atoms.

As used herein, the term "alkylene," when used alone or as part of a larger moiety (e.g., as in "*alkylene*-oxy-hetero-cyclic"), refers to a saturated aliphatic hydrocarbon group with two radical points for forming two covalent bonds with two other moieties. It can contain 1 to 12 (e.g., 1 to 8, 1 to 6, or 1 to 4) carbon atoms. As a moiety, it can be denoted as -CₙH₂ₙ-. Examples of an alkylene group include, but are not limited to, methylene (-CH₂-), ethylene (-CH₂CH₂-), and propylene (-CH₂CH₂CH₂-). When an alkylene is preceded by a carbon-number modifier, e.g., C₂₋₈, it means the alkylene group contains 2 to 8 carbon atoms.

As used herein, the term "alkynyl," when used alone or as part of a larger moiety, refers to an aliphatic hydrocarbon group with at least one triple bond. It can contain 2 to 12 (e.g., 2 to 8, 2 to 6, or 2 to 4) carbon atoms. An alkynyl group can be straight or branched. Examples of an alkynyl group include, but are not limited to, propargyl and butynyl. When an alkynyl is preceded by a carbon-number modifier, e.g., C₂₋₈, it means the alkynyl group contains 2 to 8 carbon atoms.

As used herein, the term "alkenyl," when used alone or as part of a larger moiety, refers to an aliphatic hydrocarbon group with at least one double bond. It can contain 2 to 12 (e.g., 2 to 8, 2 to 6, or 2 to 4) carbon atoms. An alkenyl group with one double bond can be denoted as -CₙH₂ₙ₋₁, or -CₙH₂ₙ₋₃ with two double bonds. Like an alkyl group, an alkenyl group can be straight or branched. Examples of an alkenyl group include, but are not limited to, allyl, isoprenyl, 2-butenyl, and 2-hexenyl. When an alkylene is preceded by a carbon-number modifier, e.g., C₃₋₈, it means the alkylene group contains 3 to 8 carbon atoms.

As used herein, the term "cycloalkyl," when used alone or as part of a larger moiety (e.g., as in *"*oxy*-cycloalky*l*"*)*,* refers to a saturated carbocyclic mono-, bi-, or tri-cyclic (fused or bridged or spiral) ring system. It can contain 3 to 12 (e.g., 3 to 10, or 5 to 10) carbon atoms. Examples of cycloalkyl groups include, but are not limited to, cyclopropyl, cyclobutyl, cyclopentyl, cyclohexyl, cycloheptyl, adamantyl, norbornyl, cubyl, octahydro-indenyl, decahydro-naphthyl, bicyclo[3.2.1]octyl, bicyclo[2.2.2]octyl, bicyclo[3.3.1]nonyl, ((aminocarbonyl)cycloalkyl)cycloalkyl. When a cycloalkyl is preceded by a carbon-number modifier, e.g., C₃₋₈, its means the alkyl group contains 3 to 8 carbon atoms.

As used herein, the term "cycloalkenyl," when used alone or as part of a larger moiety (e.g., as in *"*oxy*-cycloalkenyl"*)*,* refers to a non-aromatic carbocyclic ring system having one or more double bonds. It can contain 3 to 12 (e.g., 3 to 10, or 5 to 10) carbon atoms. Examples of cycloalkenyl groups include, but are not limited to, cyclopentenyl, 1,4-cyclohexa-di-enyl, cycloheptenyl, cyclooctenyl, hexahydro-indenyl, octahydro-naphthyl, cyclohexenyl, cyclopentenyl, bicyclo[2.2.2]octenyl, orbicyclo[3.3.1]nonenyl.

As used herein, the term "heterocycloalkyl," when used alone or as part of a larger moiety (e.g., as in "cycloalkylene-oxy-*cycloalkeny*/"), refers to a 3- to 16- membered mono-, bi-, or tri-cyclic (fused or bridged or spiral)) saturated ring structure, in which one or more of the ring atoms is a heteroatom (e.g., N, O, S, or combinations thereof). In addition to the heteroatom(s), the heterocycloalkyl can contain 3 to 15 carbon atoms (e.g., 3 to 12 or 5 to 10). Examples of a heterocycloalkyl group include, but are not limited to, piperidyl, piperazyl, tetrahydropyranyl, tetrahydrofuryl, 1,4-dioxolanyl, 1,4-dithianyl, 1,3-dioxolanyl, oxazolidyl, isoxazolidyl, morpholinyl, thiomorpholyl, octahydrobenzofuryl, octahydrochromenyl, octahydrothiochromenyl, octahydroindolyl, octahydropyrindinyl, decahydroquinolinyl, octahydrobenzo[b]thiopheneyl, 2-oxa-bicyclo[2.2.2]octyl, l-aza-bicyclo[2.2.2]octyl, 3-aza- bicyclo[3.2.1]octyl, and 2,6-dioxa-tricyclo[3.3.1.03,7]nonyl. A monocyclic heterocycloalkyl group can be fused with a phenyl moiety such as tetrahydroisoquinoline. When a heterocycloalkyl is preceded by a carbon-number modifier, e.g., C₄₋₈, it means the heterocycloalkyl group contains 4 to 8 carbon atoms.

As used herein, the term "hetero," when used alone or as part of a larger moiety (e.g., as in "heterocyclo," "heterocycloalkyl," "heterocycloalkylene" or "heteroaryl"), refers to a hetero atom or group that is -O-, -S-, or -NH-, if applicable.

As used herein, the term "aryl," when used alone or as part of a larger moiety (e.g., as in *"*alkylene*aryl"*), refers to a monocyclic (e.g., phenyl), bicyclic (e.g., indenyl, naphthalenyl, or tetrahydronaphthyl), and tricyclic (e.g., fluorenyl, tetrahydrofluorenyl, tetrahydroanthracenyl, or anthracenyl) ring system in which the monocyclic ring system is aromatic (e.g., phenyl) or at least one of the rings in a bicyclic or tricyclic ring system is aromatic (e.g., phenyl). The bicyclic and tricyclic groups include, but are not limited to, benzo-fused 2- or 3-membered carbocyclic rings. For instance, a benzo-fused group includes phenyl fused with two or more C₄₋₈ carbocyclic moieties.

As used herein, the term "heteroaryl" refers to a monocyclic, bicyclic, or tricyclic ring system having 5 to 15 ring atoms wherein at least one of the ring atoms is a heteroatom (e.g., N, O, S, or combinations thereof) and when the monocyclic ring system is aromatic or at least one of the rings in the bicyclic or tricyclic ring systems is aromatic. It can contain 5 to 12 or 8 to 10 ring atoms. A heteroaryl group includes, but is not limited to, a benzo-fused ring system having 2 to 3 rings. For example, a benzo-fused group includes benzo fused with one or two 4- to 8-membered heterocycloalkyl moieties (e.g., indolizyl, indolyl, isoindolyl, 3H-indolyl, indolinyl, benzo[b]furyl, benzo[b]thiophenyl, quinolinyl, or isoquinolinyl). Some examples of heteroaryl are pyridyl, IH-indazolyl, furyl, pyrrolyl, thienyl, thiazolyl, oxazolyl, imidazolyl, tetrazolyl, benzofuryl, isoquinolinyl, benzithiazolyl, xanthenyl, thioxanthenyl, phenothiazinyl, dihydroindolyl, benzo[1,3]dioxolyl, benzo [b] furyl, benzo [bjthiophenyl, indazolyl, benzimidazolyl, benzthiazolyl, puryl, quinolinyl, quinazolinyl, phthalazyl, quinazolyl, quinoxalyl, isoquinolinyl, 4H-quinolizyl, benzo- 1,2,5-thiadiazolyl, and 1,8-naphthyridyl.

As used herein, the suffix "-ene" is used to describe a bivalent group with two radical points for forming two covalent bonds with two other moieties. In other words, any of the terms as defined above can be modified with the suffix "-ene" to describe a bivalent version of that moiety. For example, a bivalent aryl ring structure is "arylene," a bivalent benzene ring structure is "phenylene," a bivalent heteroaryl ring structure is "heteroarylene," a bivalent cycloalkyl ring structure is a "cycloalkylene," a bivalent heterocycloalkyl ring structure is "heterocycloalkylene," a bivalent cycloalkenyl ring structure is "cycloalkenylene,"

As used herein, the term "optionally" (e.g., as in "optionally substituted with") means that the moiety at issue is either substituted or not substituted, and that the substitution occurs only when it is chemically feasible. For instance, H cannot be substituted with a substituent and a covalent bond or -C(=O)- group cannot be substituted with a substituent.

As used herein, an "oxo" or "oxide" group refers to =O.

As used herein, an "oxy" group refers to -O-.

As used herein, a "carbonyl" group refers to -C(O)- or -C(=O)-.

As used herein, the term *"*1,4-alkyl substituted piperazine" refers to

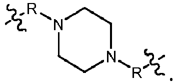

For convenience and as commonly understood, the term "optionally substituted" only applies to the chemical entities that can be substituted with suitable substituents, not to those that cannot be substituted chemically.

As used herein, the term "or" can mean *"*or*"* or *"*and.*"*

### Detailed Description of the Invention

The following examples 1-8, 10, 11, 13-16, 18-37 and 39-46 are not part of the invention and are mentioned herein for reference only.

### Example 1: Synthesis of Bis(4-isocyanatophenoxy)methane ("Isocyanate Compound 1")

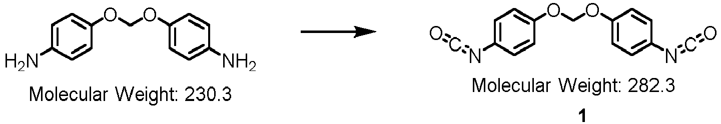

A solution of 4,4'-(methylenebis(oxy)) dianiline(23 g) in200 mL dioxane was slowly added into a solution of triphosgene (29.6g) in 300mL toluene, with the temperature controlled below 5°C.On the completion of the addition, the resultant mixture was refluxed for 4 hours. The reaction mixture was then concentrated *in vacuo,* and the residue was suspended in 500mL petroleum ether with vigorous stirring. The mixture was filtered. The filtrate was dried with anhydrous Na₂SO₄, and concentrated *in vacuo* giving 15g of Isocyanate Compound 1 as white solid with the yield of 53.2%. ¹H-NMR (CDCl₃, 400 M): 5.65 (2H, s), 7.02 (8H, s).

### Example 2: Synthesis of Isocyanate Compound 1

A solution of 4,4'-(methylenebis(oxy)) dianiline(23 g) in450 mL ethyl acetate was slowly added into a solution of triphosgene (29.6g) in150mL ethyl acetate, with the temperature controlled below 30°C.On the completion of the addition, the resultant mixture was refluxed overnight. The reaction mixture was then concentrated *in vacuo,* and the residue was suspended in 500mL petroleum ether with vigorous stirring. The mixture was filtered. The filtrate was dried with anhydrous Na₂SO₄, and concentrated *in vacuo* giving 18 g of Isocyanate Compound 1 as a white solid with the yield of 63.8%. ¹H-NMR (CDCl₃, 400 M): 5.65 (2H, s), 7.02 (8H, s).

### Example 3: Synthesis of Isocyanate Compound 1

A solution of 4,4'-(methylenebis(oxy)) dianiline(2.3 g) in20 mL dichloromethane was slowly added into a solution of triphosgene (5.92 g) in40mL dichloromethane, followed with a mixture of 6mLtriethylamine and 20mL dichloromethane. During the addition processes, the temperature gradually increased, and the reaction mixture started refluxing. On the completion of the addition, the resultant mixture was stirred at room temperature for 30 mins, and then concentrated *in vacuo.* The residue was suspended in 300mL petroleum ether with vigorous stirring. The mixture was filtered. The filtrate was dried with anhydrous Na₂SO₄, and concentrated *in vacuo* giving 1.3 g of Isocyanate Compound 1 as a white solid with the yield of 46.1%. ¹H-NMR (CDCl₃, 400 M): 5.65 (2H, s), 7.02 (8H, s).

### Example 4: Synthesis of Isocyanate Compound 1

At 0°C, a solution of 4,4'-(methylenebis(oxy)) dianiline(2.30 g) in20 mL dioxane was slowly added into a solution of triphosgene (2.96g) in 30mL dioxane, with the temperature of the mixture controlled below 30°C. Upon the completion of the addition, the resultant mixture was refluxed overnight. The reaction mixture was then concentrated *in vacuo,* and the residue was suspended in 100 mL petroleum ether with vigorous stirring. After filtering the mixture, the filtrate was dried with anhydrous Na₂SO₄ and concentrated *in vacuo* to give 1.2 g of Isocyanate Compound 1 as a white solid at the yield of 42.5%. ¹H-NMR (CDCl₃, 400 M): 5.65 (2H, s), 7.02 (8H, s).

### Example 5 (Reference Example): Synthesis of Bis(2-isocyanatoethoxy)methane (IsocyanateCompound 2)

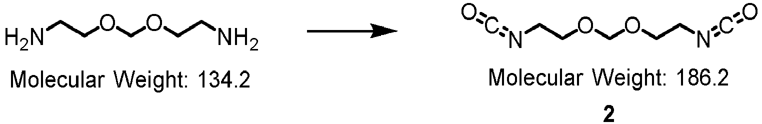

At the room temperature, a solution of 2,2'-(methylenebis(oxy)) diethanamine (1.34 g) in10 mL dichloromethane was slowly added into a solution of triphosgene (5.92 g) in40mL dichloromethane, followed with a mixture of 6 mLtriethylamine and 20 mL dichloromethane. During the above addition processes, the temperature was gradually increased, and the reaction mixture started refluxing. Upon the completion of the addition, the resultant mixture was stirred at the room temperature for 30 minutes. The resultant reaction mixture was concentrated *in vacuo,* and the residue was suspended in 100 mL petroleum ether with vigorous stirring. The mixture was filtered and the filtrate was dried with anhydrous Na₂SO₄ and concentrated *in vacuo* to give 1.1 g of Isocyanate Compound 2 as light yellow oil at the yield of 60%. ¹H-NMR (CDCl₃, 400 M) 3.50 (2H, t), 3.79(2H, t), 4.70 (2H, s).

### Example 6 (Reference Example): Synthesis of IsocyanateCompound 2

At the room temperature, a solution of 2,2'-(methylenebis(oxy)) diethanamine (1.34 g) and N, N-diisopropylethylamine(1.07mL) in 10mL dichloromethane was slowly added into a solution of triphosgene (2.96g) in20mL dichloromethane. Upon the completion of the addition, the resultant mixture was stirred at room temperature for 30 mins. The resultant reaction mixture was concentrated *in vacuo,* and the residue was suspended in 100 mL petroleum ether with vigorous stirring. Then the mixture was filtered and the filtrate was dried with anhydrous Na₂SO₄, and concentrated *in vacuo* to give 1.0 g of Isocyanate Compound 2 as light yellow oil with the yield of 53.7%. ¹H-NMR (CDCl₃, 400 M) 3.50 (2H, t), 3.79(2H, t), 4.70 (2H, s).

### Example 7 (Reference Example): Synthesis of IsocyanateCompound 2

At 0 °C, a solution of 2,2'-(methylenebis(oxy)) diethanamine (1.34 g) and pyridine (1.1 mL) in 10 mL dichloromethane was slowly added into a solution of triphosgene (2.96g) in20mL dichloromethane, with the temperature controlled below 5°C. Upon the completion of the addition, the resultant mixture was stirred at the room temperature for 30 minutes. The resultant reaction mixture was concentrated *in vacuo,* and the residue was suspended in 100 mL petroleum ether with vigorous stirring. The mixture was filtered. The filtrate was dried with anhydrous Na₂SO₄, and concentrated *in vacuo* giving 1.0 g of Isocyanate Compound 2 as light yellow oil with the yield of 53.7%. ¹H-NMR (CDCl₃, 400 M) 3.50 (2H, t), 3.79(2H, t), 4.70 (2H, s).

### Example 8 (Reference Example): Synthesis of IsocyanateCompound 2

At the room temperature, a solution of 2,2'-(methylenebis(oxy)) diethanamine (13.4 g) in 150 mL ethyl acetate was slowly added into a solution of triphosgene (29.6g) in 150mL ethyl acetate, with the temperature controlled below 30°C. On the completion of the addition, the resultant mixture was refluxed overnight. The resultant reaction mixture was concentrated *in vacuo,* and the residue was suspended in 500 mL petroleum ether with vigorous stirring. The mixture was filtered. The filtrate was dried with anhydrous Na₂SO₄, and concentrated *in vacuo* giving 12 g of Isocyanate Compound 2 as light yellow oil with the yield of 63.8%. ¹H-NMR (CDCl₃, 400 M) 3.50 (2H, t), 3.79(2H, t), 4.70 (2H, s).

### Example 9 (according to the invention): Synthesis of IsocyanateCompound 3

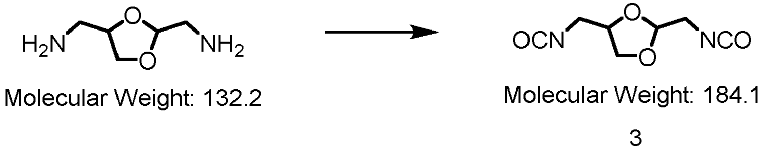

At the room temperature, a solution of (1,3-dioxolane-2,4-diyl)dimethanamine (2g) in 10 mL dichloromethane was slowly added into a solution of triphosgene (10g) in80mL dichloromethane, followed with a mixture of 6 mL of triethylamine and 20 mL of dichloromethane. During the above addition processes, the temperature gradually increased, and the reaction mixture started refluxing. On the completion of the addition, the resultant mixture was stirred at room temperature overnight. The resultant reaction mixture was concentrated *in vacuo,* and the residue was suspended in 100 mL petroleum ether with vigorous stirring. The mixture was filtered, and the filtrate was dried with anhydrous Na₂SO₄ and then concentrated *in vacuo* to give 1.7 g of Isocyanate Compound 3 as light yellow oil with the yield of 61%.

### Example 10: Preparation of degradable polyurethane

Polyethyleneglycol 1000 and Isocyanate Compound 1 were mixed at the mass ratio of 100/28.2. After quickly defoamed under vacuumwith vigorous stirring, the mixture was cured at the room temperature, followed by postcured in an 80 °C oven for 2 hours to give a degradable polyurethane.

### Example 11 (Reference Example): Preparation of degradable polyurethane

Polyethyleneglycol 1000 and Isocyanate Compound 2 were mixed at the mass ratio of 100/18.6. After quickly defoamed under vacuumwith vigorous stirring, the mixture was cured at the room temperature, followed by postcured in an 80 °C oven for 2 hours to give a degradable polyurethane.

### Example 12 (according to the invention): Preparation of degradble polyurethane

Polyethyleneglycol 1000 and Isocyanate Compound 3 were mixed at the mass ratio of 100/18.4. After quickly defoamed under vacuumwith vigorous stirring, the mixture was cured at room temperature, followed by postcured in an 80 °C oven for 2 hours to give a degradable polyurethane.

### Example 13: Degradation of degradable polyurethane

In a round-bottomed flask, a piece of the degradable polyurethane sample (1.0 g) from Example 10 was imerged in a mixture of 10 mL concentrated hydrochloric acid and 90 mL ethylene glycol. The degradation solution was stirred at 100 °C for 4 hours to give a clear solution which was neutralized with a 20% aqueous sodium hydroxide solution. The resultant suspension was filtered, and the collected solid was washed with water and dried, giving a mass recovery yield of 96.5%.

### Example 14: Degradation of degradable polyurethane

In a round-bottomed flask, a piece of the degradable polyurethane sample (1.0 g) from Example 10 was imerged in a mixture of 1 mL concentrated hydrochloric acid and 90 mL ethylene glycol. The degradation solution was stirred at 180 °C for 2 hours to give a clear solution which was neutralized with a 20% aqueous sodium hydroxide solution. The resultant suspension was filtered, and the collected solid was washed with water and dried, giving a mass recovery yield of 96%.

### Example 15 (Reference Example): Degradation of degradable polyurethane

In a round-bottomed flask, a piece of the degradable polyurethane sample (1.0 g) from Example 11 was imerged in a mixture of 10 mL concentrated hydrochloric acid and 90 mL ethylene glycol. The degradation solution was stirred at 100 °C for 4 hours giving a clear solution which was neutralized with a 20% aqueous sodium hydroxide solution. The resultant suspension was filtered, and the collected solid was washed with water and dried, giving a mass recovery yield of 97%.

### Example 16 (Reference Example): Degradation of degradable polyurethane

In a round-bottomed flask, a piece of degradable polyurethane sample (1.0 g) from Example 11 was imerged in a mixture of 1 mL concentrated hydrochloric acid and 90 mL ethylene glycol. The degradation solution was stirred at 180°C for 2 hours giving a clear solution which was neutralized with a 20% aqueous sodium hydroxide solution. The resultant suspension was filtered, and the collected solid was washed with water and dried, giving a mass recovery yield of 98%.

### Example 17 (according to the invention): Degradation of degradable polyurethane

In a round-bottomed flask, a piece of the degradable polyurethane sample (1.0 g) from Example 12 was imerged in a mixture of 1 mL concentrated hydrochloric acid and 90 mL ethylene glycol. The degradation solution was stirred at 140 °C for 1 hour to give a clear solution which was neutralized with a 20% aqueous sodium hydroxide solution. The resultant suspension was filtered, and the collected solid was washed with water and dried, giving a mass recovery yield of 98%.

### Example 18: Preparation of recyclablecarbon fibre polyurethane composite

Polyethyleneglycol 1000 and Isocyanate Compound 1 were mixed at the mass ratio of 100/28.2. After quickly defoamed under vacuumwith vigorous stirring, the mixture was evenly applied over three layers of 2x2 twill carbon fiber (3K) fabric sheets. The resultant stack was then cured on a flat hot-pressing machine at 80 °C under a pressure of 10 atms for 2 hours, giving a recyclable carbon fiber polyurethane composite laminate.

### Example 19 (Reference Example): Preparation of recyclablecarbon fibre polyurethane composite

Polyethyleneglycol 1000 and isocyanate II were mixed at the mass ratio of 100/18.6. After quickly defoamed under vacuumwith vigorous stirring, the mixture was evenly applied over three layers of 2x2 twill carbon fiber (3K) fabric sheets. The resultant stack was then cured on a flat hot-pressing machine at 80 °C under a pressure of 10 atms for 2 hours, giving a recyclable carbon fiber polyurethane composite laminate.

### Example 20 (Reference Example): Preparation of recyclablecarbon fibre polyurethane composite

Polyethyleneglycol 1000 and isocyanate III were mixed at the mass ratio of 100/18.4. After quickly defoamed under vacuumwith vigorous stirring, the mixture was evenly applied over three layers of 2x2 twill carbon fiber (3K) fabric sheets. The resultant stack was then cured on a flat hot-pressing machine at 80 °C under a pressure of 10 atms for 2 hours giving a recyclable carbon fiber polyurethane composite laminate.

### Example 21: Degradation of degradable polyurethane in recyclable carbon fiber polyurethane composite panel

In a round-bottomed flask, a piece of recyclable carbon fiber polyurethane composite sample (1.0 g) from Example 18 was imerged in a mixture of 10 mL concentrated hydrochloric acid and 90 mL ethylene glycol. After heated at 100°C for 4 hours, the degradation solution was filtered to separate the carbon fibres, and the filtrate was neutralized with a 20% aqueous sodium hydroxide solution. The resultant suspension was filtered again, and the collected solid was washed with water and dried, giving a mass recovery yield of 96%.

### Example 22: Degradation of degradable polyurethane in recyclable carbon fiber polyurethane composite panel

In a round-bottomed flask, a piece of recyclable carbon fiber polyurethane composite sample (1.0 g) from Example 18 was imerged in a mixture of 1 mL concentrated hydrochloric acid and 90 mL ethylene glycol. After heated at 180 °C for 2 hours, the degradation solution was filtered to separate the carbon fibres, and the filtrate was neutralized with a 20% aqueous sodium hydroxide solution. The resultant suspension was filtered again, and the collected solid was washed with water and dried, giving a mass recovery yield of 96%.

### Example 23 (Reference Example): Degradation of degradable polyurethane in recyclable carbon fiber polyurethane composite panel

In a round-bottomed flask, a piece of recyclable carbon fiber polyurethane composite sample (1.0 g) from Example 19 was imerged in a mixture of 10 mL concentrated hydrochloric acid and 90 mL ethylene glycol. After heated at 120 °C for 2 hours, the degradation solution was filtered to separate the carbon fibres, and the filtrate was neutralized with a 20% aqueous sodium hydroxide solution. The resultant suspension was filtered again, and the collected solid was washed with water and dried, giving a mass recovery yield of 97%.

### Example 24 (Reference Example): Degradation of degradable polyurethane in recyclable carbon fiber polyurethane composite panel

In a round-bottomed flask, a piece of recyclable carbon fiber polyurethane composite sample (1.0 g) from Example 19 was imerged in a mixture of 5mL concentrated hydrochloric acid and 90mL ethylene glycol. After heated at 150 °C for 4 hours, the degradation solution was filtered to separate the carbon fibres, and the filtrate was neutralized with a 20% aqueous sodium hydroxide solution. The resultant suspension was filtered again, and the collected solid was washed with water and dried, giving a mass recovery yield of 98%.

### Example 25 (Reference Example): Degradation of degradable polyurethane in recyclable carbon fiber polyurethane composite panel

In a round-bottomed flask, a piece of recyclable carbon fiber polyurethane composite sample (1.0 g) from Example 20 was imerged in a mixture of 5mL concentrated hydrochloric acid and 90mL ethylene glycol. After heated at 160 °C for 6 hours, the degradation solution was filtered to separate the carbon fibres, and the filtrate was neutralized with a 20% aqueous sodium hydroxide solution. The resultant suspension was filtered again, and the collected solid was washed with water and dried, giving a mass recovery yield of 97%.

### Example 26: Degradable cross-linked polymer

In a round-bottomed flask, degradable Isocyanate Compound 1 (2.8g) and liquid bisphenolA type epoxy resin E52D (EEW 188.5g/eq, 18.9g) were stirred at 120°C for 2 hours. The resultant product was then mixed with degradable curing agent 2,2'-(methylenebis(oxy)) diethanamine (AEW 33.5 g/eq, 3.4g,) at room temperature, and the mixture was cured at 120 °C for 2 hours giving the degradable polymer.

### Example 27 (Reference Example): Degradable polymer

At the room temperature and in a round-bottomed flask, degradable Isocyanate Compound 2 (2.8g), liquid bisphenolA type epoxy resin E52D (EEW 188.5g/eq, 18.9g), and degradable curing agent 4,4'-(methylenebis(oxy)) dianiline (AEW 57.5g/eq, 5.8g,) were mixed and stirred. The resultant mixture was then spread on a panel and cured at 120 °C for 2 hours, giving a degradable cross-linked polymer.

### Example 28 (Reference Example): Degradable polymer

At the room temperature and in a round-bottomed flask, degradable isocyanate III (2.7g), liquid bisphenolA type epoxy resin E52D (EEW 188.5g/eq, 18.9g), and degradable curing agent 4,4'-(methylenebis(oxy)) dianiline (AEW 57.5g/eq, 5.8g,). The resultant mixture was then spread on a panel and cured at 120 °C for 2 hours giving the degradable polymer.

### Example 29: Degradation of the degradable polymer

In a round-bottomed flask, a piece of degradable polymer sample (0.5 g) from Example 26 was imerged in a mixture of 10 mL concentrated hydrochloric acid and 90mL ethylene glycol. The degradation solution was stirred at 180 °C for 10 hours giving a clear solution which was neutralized with a 20% aqueous sodium hydroxide solution. The resultant suspension was filtered, and the collected solid was washed with water and dried giving 0.48g of degraded polymer of mass recovery yield of 96%.

### Example 30: Degradation of the degradable polymer

In an autoclave, a piece of degradable polymer sample (0.7 g) from Example 26 was treated in a mixture of 0.1 mL concentrated hydrochloric acid and 90mL ethylene glycol. The degradation solution was stirred at 350 °C for 0.5 hours. The resultant clear solution was transferred into a clean beaker, and neutralized with a 20% aqueous sodium hydroxide solution. The resultant suspension was filtered, and the collected solid was washed with water and dried giving 0.66g of degraded polymer of mass recovery yield of 94%.

### Example 31: Degradation of the degradable polymer

In a round-bottomed flask, a piece of degradable polymer sample (0.4 g) from Example 26 was imerged in a mixture of 30 mL concentrated hydrochloric acid and 70 mL ethylene glycol. The degradation solution was stirred at room temperature for 120 hours giving a clear solution which was neutralized with a 20% aqueous sodium hydroxide solution. The resultant suspension was filtered, and the collected solid was washed with water and dried giving 0.38g of degraded polymer of mass recovery yield of 95%.

### Example 32 (Reference Example): Degradation of the degradable polymer

In a round-bottomed flask, a piece of degradable polymer sample (0.7 g) from Example 27 was imerged in a mixture of 10 mL concentrated hydrochloric acid and 90mL ethylene glycol. The degradation solution was stirred at 190 °C for 4 hours giving a clear solution which was neutralized with a 20% aqueous sodium hydroxide solution. The resultant suspension was filtered, and the collected solid was washed with water and dried giving 0.68g of degraded polymer of mass recovery yield of 97%.

### Example 33 (Reference Example): Degradation of the degradable polymer

In a round-bottomed flask, a piece of degradable polymer sample (0.6g) from Example 27 was imerged in a mixture of 10mL concentrated hydrochloric acid and 45mL benzyl alcohol. The degradation solution was stirred at 190°C for 4 hours giving a clear solution which was neutralized with a 20% aqueous sodium hydroxide solution. The resultant suspension was filtered, and the collected solid was washed with water and dried giving 0.57g of degraded polymer of mass recovery yield of 95%.

### Example 34 (Reference Example): Degradation of the degradable polymer

In a round-bottomed flask, a piece of degradable polymer sample (0.6g) from Example 28 was imerged in a mixture of 10mL concentrated hydrochloric acid and 90mL octanol. The degradation solution was stirred at 120°C for 8 hours giving a clear solution which was neutralized with a 20% aqueous sodium hydroxide solution. The resultant suspension was filtered, and the collected solid was washed with water and dried giving 0.58g of degraded polymer of mass recovery yield of 96%.

### Example 35 (Reference Example): Degradation of the degradable polymer

In a round-bottomed flask, a piece of degradable polymer sample (0.63g) from Example 28 was imerged in a mixture of 10mL concentrated hydrochloric acid and 90mL octanol. The degradation solution was stirred at 155°C for 4 hours giving a clear solution which was neutralized with a 20% aqueous sodium hydroxide solution. The resultant suspension was filtered, and the collected solid was washed with water and dried giving 0.6g of degraded polymer of mass recovery yield of 95%.

### Example 36: Preparation ofrecyclable carbon fiber composite laminate

At the room temperature and in a round-bottomed flask, degradable Isocyanate Compound 1 (28g) and liquid bisphenolA type epoxy resin E52D (EEW 188.5g/eq, 189 g) were stirred at 120°C for 2 hours. The resultant product was further mixed with degradable curing agent 2,2'-(methylenebis(oxy)) diethanamine (AEW 33.5g/eq, 34 g,) at the room temperature, and the mixture was then quickly defoamed under vacuum with vigorous stirring. It was then cooled to the room temperature and then stored in a freezer.

After being heated to 50 °C, the resultant resin mixture was evenly applied over five layers of 2x2 twill carbon fiber (3K) fabric sheets. And the stack was then cured on a flat hot-pressing machine at 150 °C under a pressure of 10 atms for 2 hours, giving a recyclable carbon fiber composite laminate.

### Example 37 (Reference Example): Preparation for recyclable carbon fiber composite laminate

At the room temperature and in a round-bottomed flask, degradable Isocyanate Compound 2 (28g), liquid bisphenolA type epoxy resin E52D (EEW 188.5g/eq, 189 g) were stirred at 120°C for 2 hours. The resultant product was further mixed with degradable curing agent 4,4'-(methylenebis(oxy)) dianiline (AEW 57.5g/eq, 58g,) at the room temperature on a three roll grinder, and the mixture was then quickly defoamed under vacuum in a high speed mixer, cooled to the room temperature and then stored in a freezer.

Carbon fibre prepreg was prepared by impregnating 2x2 twill carbon fiber (3K) fabric sheet with the above prepared degradable epoxyresin at 50 °C. A stack of five layers of the prepared carbon fiber prepreg was cured on a flat hot-pressing machine at 150 °C under a pressure of 10 atms for 2 hours, giving a recyclable carbon fiber composite laminate.

### Example 38 (according to the invention): Preparation for recyclable carbon fiber composite laminate

At the room temperature and in a round-bottomed flask, degradable Isocyanate Compound3 (27.8g) and liquid bisphenolA type epoxy resin E52D (EEW 188.5g/eq, 189g) were stirred at 120°C for 2 hours. The resultant product was further mixed with degradable curing agent 4,4'-(methylenebis(oxy)) dianiline (AEW 57.5g/eq, 58 g,) at the room temperature on a three roll grinder, and the mixture was then quickly defoamed under vacuum in a high speed mixer and stored in a freezer.

Carbon fibre prepreg was prepared by impregnating 2x2 twill carbon fiber (3K) fabric sheet with the above prepared degradable epoxyresin at 50 °C. A stack of five layers of the prepared carbon fiber prepreg was cured on a flat hot-pressing machine at 150 °C under a pressure of 10 atms for 2 hours giving a recyclable carbon fiber composite laminate.

### Example 39: Degradation of recyclable carbon fiber composite laminate

In a round-bottomed flask, a piece of recyclable carbon fiber composite laminate sample (1.6 g) from Example 36 was imerged in a mixture of 10 mL concentrated hydrochloric acid and 90 mL benzyl alcohol. After heated at 190 °C for 3 hours, the degradation solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 1.55 g, giving a mass recovery yield of 97%. The surface of the recycled carbon fiber was very clean and did not have any sign of damage.

### Example 40: Degradation of recyclable carbon fiber composite laminate

In a round-bottomed flask, a piece of recyclable carbon fiber composite laminate sample (1.5 g) from Example 36 was imerged in a mixture of 10 mL concentrated hydrochloric acid and 90 mL ethylene glycol. After heated at 160 °C for 3 hours, the degradation was almost complete and the solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 1.46 g, giving a mass recovery yield of 97%. The surface of the recycled carbon fiber was very clean and essentially free of signs of damage.

### Example 41: Degradation of recyclable carbon fiber composite laminate

In a round-bottomed flask, a piece of recyclable carbon fiber composite laminate sample (1.3 g) from Example 36 was imerged in a mixture of 10mL concentrated hydrochloric acid and 90mL hexanol. After heated at 135°C for 4 hours, the degradation was almost complete and the solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 1.23 g, giving a mass recovery yield of 95%. The surface of the recycled carbon fiber was very clean and essentially free of signs of damage.

### Example 42: Degradation of recyclable carbon fiber composite laminate

In a round-bottomed flask, a piece of recyclable carbon fiber composite laminate sample (1.0 g) from Example 36 was imerged in a mixture of 10 mL concentrated hydrochloric acid and 90 mL octanol. After heated at 135°C for 4 hours, the degradation was almost complete and the solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 0.96 g, giving a mass recovery yield of 96%. The surface of the recycled carbon fiber was very clean and essentially free of signs of damage.

### Example 43 (Reference Example): Degradation of cyclable carbon fiber composite laminate

In a round-bottomed flask, a piece of recyclable carbon fiber composite laminate sample (0.9g) from Example 37 was imerged in a mixture of 10mL concentrated hydrochloric acid and 90mL ethylene glycol. After heated at 135°C for 4 hours, the degradation was almost complete and the solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 0.86 g, giving a mass recovery yield of 95%. The surface of the recycled carbon fiber was very clean and essentially free of signs of damage.

### Example 44 (Reference Example): Degradation of recyclable carbon fiber composite laminate

In a round-bottomed flask, a piece of recyclable carbon fiber composite laminate sample (1.1g) from Example 37 was imerged in a mixture of 5mL concentrated hydrochloric acid and 90mL ethylene glycol. After heated at 185°C for 3 hours, the degradation was almost complete and the solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 0.86 g, giving a mass recovery yield of 95%.

### Example 45 (Reference Example): Degradation of recyclable carbon fiber composite laminate

In a round-bottomed flask, a piece of recyclable carbon fiber composite laminate sample (2g) from Example 37 was imerged in a mixture of 5mL methanesulfonic acid and 90mL octanol. After heated at 160°C for 3 hours, the degradation was almost complete and the solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 1.94 g, giving a mass recovery yield of 97%. The surface of the recycled carbon fiber was very clean and essentially free of signs of damage.

### Example 46 (Reference Example): Degradation of recyclable carbon fiber composite laminate

In a round-bottomed flask, a piece of recyclable carbon fiber composite laminate sample (1.0 g) from Example 37 was imerged in a mixture of 5mL methanesulfonic acid and 90mL hexanol. After heated at 135°C for 4 hours, the degradation was almost complete and solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 0.95 g, giving a mass recovery yield of 95%. The surface of the recycled carbon fiber was very clean and essentially free of signs of damage.

### Example 47 (according to the invention): Degradation of recyclable carbon fiber composite laminate

In an autoclave, a piece of recyclable carbon fiber composite laminate sample (0.6g) from Example 38 was imerged in a mixture of 0.1 mL concentrated hydrochloric acid and 90 mL ethylene glycol. After heated at 350°C for 0.5 hours, the degradation was almost complete and the solution was filtered to separate the carbon fibres, and the filtrate was neutralized with 2% aqueous sodium hydroxide solution. The resultant suspension was filtered again, and the collected solid was washed with water and dried. The total mass of recovered carbon fibre and degraded resin was 0.57 g, giving a mass recovery yield of 95%. The surface of the recycled carbon fiber was very clean and essentially free of signs of damage.

### Example 48 (according to the invention): Degradation of recyclable carbon fiber composite laminate

In a round-bottomed flask, a piece of recyclable carbon fiber composite laminate sample (1.0 g) from Example 38 was imerged in a mixture of 30mL concentrated hydrochloric acid and 70 mL ethylene glycol. After treated at room temperature for 120 hours, the degradation was almost complete and the solution was filtered to separate the carbon fibres, and the filtrate was neutralized with a 20% aqueous sodium hydroxide solution. The resultant suspension was filtered again, and the collected solid was washed with water and dried. The total mass of recovered carbon fibre and degraded resin was 0.95 g, giving a mass recovery yield of 95%.

## Claims

1. An isocyanate compound of Formula (I): 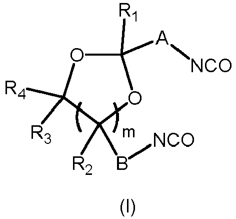 wherein,
m is 1, 2, 3, 4, or 5;
each of R₁, R₂, R₃ and R₄ independently is hydrogen, alkyl, cycloalkyl, heterocyclic, heterocyclic, alkenyl, cycloalkenyl, alkynyl, aryl, heteroaryl, alkylene-oxy-alkyl, alkylene-oxy-alkyl, alkylene-oxy-hetero-cyclic, alkylene-oxy-hetero-cycloalkyl, alkylene-oxy-alkenyl, alkylene-oxy-cycloalkenyl, alkylene-aryl, alkylene-oxy-heteroaryl, cycloalkylene-oxy-alkyl, cycloalkylene-oxy-cycloalkyl, cycloalkylene-oxy-heterocyclic, cycloalkylene-oxy-heterocycloalkyl, cycloalkylene-oxy-alkenyl, cycloalkylene-oxy-cycloalkenyl, cycloalkylene-oxy-aryl, cycloalkylene-oxy-heteroaryl, heterocycloalkylene-oxy-alkyl, heterocycloalkylene-oxy-cycloalkyl, heterocycloalkylene-oxy-heterocyclic, heterocycloalkylene-oxy-heterocycloalkyl, heterocycloalkylene-oxy-alkenyl, heterocycloalkylene-oxy-cycloalkenyl, heterocycloalkylene-oxy-aryl, heterocycloalkylene-oxy-heteroaryl, arylene-oxy-alkyl, arylene-oxy-cycloalkyl, arylene-oxy-heterocyclic, arylene-oxy-heterocycloalkyl, arylene-oxy-alkenyl, arylene-oxy-cycloalkenyl, arylene-oxy-aryl, or arylene-oxy-heteroaryl; or
R₃ and R₄, together with the carbon atom to which they are bonded, form a 3-7 membered ring optionally containing one or more heteroatoms each of which is independently S, O, or N; or
R₁ and A; together with the carbon atom to which they are bonded, form a 3-7 membered ring optionally containing one or more heteroatoms each of which is independently S, O, or N; or
R₂ and B, together with the carbon atom to which they are bonded, form a 3-7 membered ring optionally containing one or more heteroatoms each of which is independently S, O, or N; or
each of A and B independently is alkylene, alkylene-hetero-alkylene, alkenylene, alkenylene-hetero-alkenylene, alkylene-hetero-alkenylene, alkynylene, cycloalkylene, alkylene-cycloalkylene, alkylene-cycloalkylene-alkylene, alkenylene-cycloalkylene, alkenylene-cycloalkylene-alkenylen, alkylene-cycloalkylene-alkenylene, alkynylene-cycloalkylene, alkynylene-cycloalkylene-alkynylene, heterocycloalkylene, alkylene-heterocycloalkylene, alkylene-heterocycloalkylene-alkylene, alkenylene-heterocycloalkylene, alkenylene-heterocycloalkylene-alkenylene, alkylene-heterocycloalkylene-alkenylene, alkynylene-heterocycloalkylene, alkynylene-heterocycloalkylene-alkynylene, cycloalkenylene, alkylene-cycloalkenylene, alkylene-cycloalkenylene-alkylene, alkenylene-cycloalkenylene, alkenylene-cycloalkenylene-alkenylene, alkylene-cycloalkenylene-alkenylene, alkynylene-cycloalkenylene, alkynylene-cycloalkenylene-alkynylene, heterocycloalkenylene, alkylene-heterocycloalkenylene, alkylene-heterocycloalkenylene-alkylene, alkenylene-heterocycloalkenylene, alkenylene-heterocycloalkenylene-alkenylene, alkylene-heterocycloalkenylene-alkenylene, alkynylene-heterocycloalkenylene, alkynylene-heterocycloalkenylene-alkynylene, Arylene, alkylene-arylene, alkylene-arylene-alkylene, alkenylene-arylene, alkenylene-arylene-alkenylene, alkylene-arylene-alkenylene, alkynylene-arylene, alkynylene-arylene-alkynylene, Heteroarylene, alkylene-heteroarylene, alkylene-heteroarylene-alkylene, alkenylene-heteroarylene, alkenylene-heteroarylene-alkenylene, alkylene-heteroarylene-alkenylene, alkynylene-heteroarylene, alkynylene-heteroarylene-alkynylene, carbonyl, or thiocarbonyl;
wherein, optionally, m is 1; each of R₁, R₂, R₃ and R₄ is independently hydrogen or alkyl; or each A and B independently is alkylene or alkenylene;
and wherein alkyl, when used alone or as part of a larger moiety, can be substituted with one or more substituents.

2. The isocyanate compound of claim 1, wherein the isocyanate is 2,4-bis(isocyanatomethyl)-1,3-dioxolane.

3. A method for preparing an isocyanate compound of any of claims 1-2, comprising the step of converting a compound of Formula (I-A) to the isocyanate compound, wherein R, R₁, R₂, R₃, R₄, A, B and m are the same as those in the isocyanate compound.

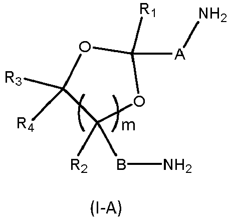

4. The method of claim 3, wherein the conversion of the compound of Formula (I-A) to the isocyanate compound is by reacting the compound of Formula (I-A) with phosgene, triphosgene, or trichloronethyl chloroformate, optionally at the presence of a catalyst; wherein, optionally, the molar ratio of the compound of Formula (I-A) to phosgene, triphosgene, or trichloronethyl chloroformate is 1:2^{∼}100, the reaction temperature is in the range of -20^{∼}150 °C; and the catalyst, when present, comprises an amine, a pyridine derivative, or N,N-dimethyl formamide.

5. A method for preparing an isocyanate compound of any of claims 1-2, comprising the step of converting a compound of Formula (I-B) to the isocyanate compound, wherein R, R₁, R₂, R₃, R₄, A, B and m are the same as those in the isocyanate compound, and each X is independently hydroxyl, thiol, or trimethylsiloxy

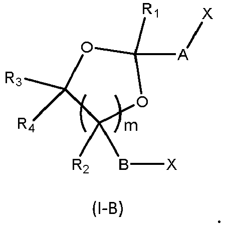

6. The method of claim 5, wherein the conversion of the compound of Formula (I-B) to the isocyanate compound is by reacting the compound of Formula (I-B) with tetrabutylammonium cyanate, optionally with the presence of a catalyst;
wherein, optionally, the molar ratio of the compound of Formula (I-B) to tetrabutylammonium cyanate is 1:2^{∼}100, the reaction temperature is in the range of-20^{∼}150 °C; and the catalyst, when present, comprises a triazine compound.

7. A degradable polyurethane, wherein the polyurethane is made by polymerizing an isocyanate compound of any of claims 1-2 with a hydrogen-donating compound which comprises a dihydric alcohol, a polyhydric alcohol, polyetherpolyol, polyesterpolyol, binary mercaptan, polybasic mercaptan, phenol, carboxylic acid, urea, amide, diamine, or polyamine; and the polyurethane has a cleavable cross-linking structure of Formula (I-C), wherein R, R₁, R₂, R₃, R₄, A, B and m are the same as those in the isocyanate compound

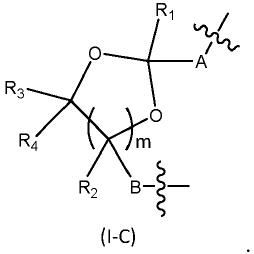

8. A degradable cross-linked polymer, wherein the polymer is made by polymerizing an isocyanate compound of any of claims 1-2 with an epoxy resin and a degradable curing agent; the epoxy resin comprises a glycidyl ether epoxy resin, a glycidyl ester epoxy resin, glycidyl epoxy amine epoxy resin, a trifunctional epoxy resin, a tetrafunctional epoxy resin, a novolac epoxy resin, an o-cresol formaldehyde epoxy resin, an aliphatic epoxy resin, an alicyclic epoxy resin, or a nitrogen-containing epoxy resin; and the degradable curing agent comprises an acetal or ketal aliphatic amine, an acetal or ketal aromatic amine or salt thereof, an acetal or ketal polyamine, a cyclic acetal or ketal amine, an acetal or ketal hydrazide, or hydrazone; and the cross-linked polymer has a cleavable cross-linking structure of Formula (I-C), wherein R, R₁, R₂, R₃, R₄, A, B and m are the same as those in the isocyanate compound

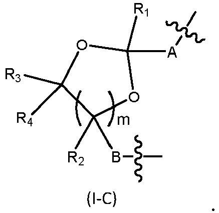

9. A method for degrading a polyurethane of claim 7 or a cross-linked polymer of claim 8, comprising the following steps:
(1) under the heating and stirring conditions, the degradable polyurethane or the degradable cross-linked polymer is immersed in a mixed acid and solvent system for the degradation for 1^{∼}600 hours at a temperature within the range of 15^{∼}400 °C, wherein the mass concentration of acid in the solvent 0.1^{∼}99%;
(2) using an alkali solution to adjust the pH of the degradation solution to above 6 at a temperature within the range of 0°^{∼}200 °C, wherein the mass concentration of alkali solution is 0.1^{∼}99%.

10. The method of claim 9, wherein the acid comprises hydrochloric acid, hydrobromic acid, hydrofluoric acid, acetic acid, trifluoroacetic acid, lactic acid, formic acid, propionic acid, citric acid, methanesulfonic acid, p-toluenesulfonic acid, nitric acid, sulfuric acid, sulfurous acid, phosphoric acid, perchloric acid, benzoic acid, salicylic acid, or phthalic acid; the solvent system comprises methanol, ethanol, ethylene glycol, propanol, isopropanol, butanol, isobutanol, t-butanol, pentanol, hexanol, heptanol, octanol, nonanol, benzyl alcohol, phenethyl alcohol, p-hydroxymethyl benzene, m-hydroxymethyl benzene, o-hydroxy benzene, p-hydroxyethyl benzene, m-hydroxyethyl benzene, o-hydroxyethyl benzene, water, N,N-dimethylformamide, N,N-dimethylacetamide, N-methylpyrrolidone, dimethyl sulfoxide, tetrahydrofuran, methyl tetrahydrofuran, glycerol, or dioxane; the alkali comprises lithium hydroxide, sodium hydroxide, potassium hydroxide, calcium hydroxide, sodium carbonate, sodium bicarbonate, potassium carbonate, potassium bicarbonate, or ammonia; the solvent of the alkali solution comprises methanol, ethanol, ethylene glycol, propanol, isopropanol, butanol, isobutanol, t-butanol, pentanol, hexanol, heptanol, octanol, nonanol, benzyl alcohol, phenethyl alcohol, p-hydroxymethyl benzene, m-hydroxymethyl benzene, o-hydroxy benzene, p-hydroxyethyl benzene, m-hydroxyethyl benzene, o-hydroxyethyl benzene, water, N,N-dimethylformamide, N,N-dimethylacetamide, N-methylpyrrolidone, dimethyl sulfoxide, tetrahydrofuran, methyl tetrahydrofuran, glycerol, or dioxane;
and / or wherein in step (1), the heating temperature is within the range of 80^{∼}150 °C, the heating time is within the range of 4^{∼}8 hours, and the mass concentration of acid in the solvent 0.5^{∼}20%; in step (2), the temperature is within the range of 5^{∼}50 °C, the final pH value after adjustment with the alkali solution is in the range of 6^{∼}12, and the mass concentration of alkali solution is within the range of 5^{∼}30%.

11. A recyclable reinforced composite material comprising a polyurethane of claim 7 or a cross-linked polymer of claim 8, a reinforcing material, and an auxiliary material, wherein the reinforcing material comprises carbon nanotubes, boron nitride nanotubes, carbon black, metal nano-particles, metal oxide nanoparticles, organic nanoparticles, iron oxide, glass fibers, carbon fibers, natural fibers, synthetic fibers and fabric made therefrom; and the auxiliary material comprises an accelerator, a diluent, a plasticizer, a toughening agent, a thickening agent, a coupling agent, a defoamer, a flatting agent, an ultraviolet absorber, an antioxidant, a brightener, a fluorescent agent, a pigment, or a filler.

12. A method for recycling a reinforced composite material of claim 11, comprising the steps of:
(1) under the heating and stirring conditions, immersing the reinforced composite material in a solution comprising an acid and a solvent and then heating the mixture at a temperature within the range of 15^{∼}400 °C for 1^{∼}600 hours to give rise to a degradation solution, wherein the mass concentration of acid in the solution is 0.1^{∼}99%;
(2) using an alkali solution of 0^{∼}200°C to adjust the pH value of the degradation solution from step (1) to be greater than 6 to obtain a precipitate, wherein the mass concentration of the alkali in the alkali solution is 0.1^{∼}99%;
(3) separate, wash and dry the precipitate obtained in step (2);
wherein, optionally, the acid comprises hydrochloric acid, hydrobromic acid, hydrofluoric acid, acetic acid, trifluoroacetic acid, lactic acid, formic acid, propionic acid, citric acid, methanesulfonic acid, p-toluenesulfonic acid, nitric acid, sulfuric acid, sulfurous acid, phosphoric acid, perchloric acid, benzoic acid, salicylic acid, or phthalic acid; the solvent comprises at least one of methanol, ethanol, ethylene glycol, propanol, isopropanol, butanol, isobutanol, t-butanol, pentanol, hexanol, heptanol, octanol, nonanol, benzyl alcohol, phenethyl alcohol, p-hydroxymethyl benzene, m-hydroxymethyl benzene, o-hydroxy benzene, p-hydroxyethyl benzene, m-hydroxyethyl benzene, o-hydroxyethyl benzene, water, N,N-dimethylformamide, N,N-dimethylacetamide, N-methylpyrrolidone, dimethyl sulfoxide, tetrahydrofuran, methyl tetrahydrofuran, glycerol, or dioxane; the alkali comprises lithium hydroxide, sodium hydroxide, potassium hydroxide, calcium hydroxide, sodium carbonate, sodium bicarbonate, potassium carbonate, potassium bicarbonate, or ammonium hydroxide; and the alkali solvent comprises methanol, ethanol, ethylene glycol, propanol, isopropanol, butanol, isobutanol, t-butanol, pentanol, hexanol, heptanol, octanol, nonanol, benzyl alcohol, phenethyl alcohol, p-hydroxymethyl benzene, m-hydroxymethyl benzene, o-hydroxy benzene, p-hydroxyethyl benzene, m-hydroxyethyl benzene, o-hydroxyethyl benzene, water, N,N-dimethylformamide, N,N-dimethylacetamide, N-methylpyrrolidone, dimethyl sulfoxide, tetrahydrofuran, methyl tetrahydrofuran, glycerol, or dioxane;
wherein, further optionally, in step (1), the mass concentration of acid in the solvent is within the range of 0.5^{∼}20%, the temperature is within the range of 80^{∼}200 °C, and the reaction time is 2^{∼}12 hours; and in step (2), the mass concentration of alkali solution is within the range of 5^{∼}30%, the temperature is within the range of 5^{∼}60 °C.

## Patentansprüche

1. Eine Isocyanatverbindung der Formel (I) 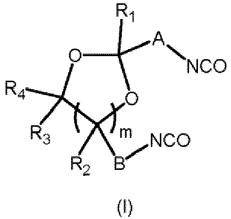 wobei
m gleich 1, 2, 3, 3, 4 oder 5 ist;
jedes von R₁, R₂, R₃ und R₄ unabhängig Wasserstoff, Alkyl, Cycloalkyl, heterocyclisch, heterocyclisch, Alkenyl, Cycloalkenyl, Alkinyl, Aryl, Heteroaryl, Alkylen-Oxy-Alkyl, Alkylen-Oxy-Alkyl, Alkylen-Oxy-Alkyl, Alkylen-Oxy-Hetero-Cycloalkyl, Alkylen-Oxy-Hetero-Cycloalkyl ist, Alkylen-Oxyalkenyl, Alkylen-Oxy-Cycloalkenyl, Alkylen-Aryl, Alkylen-Oxy-Heteroaryl, Cycloalkylen-Oxy-Alkyl, Cycloalkylen-Oxy-Cycloalkyl, Cycloalkylen-Oxy-Heterocyclen, Cycloalkylen-Oxy-Heterocycloalkyl, Cycloalkylen-Oxy-Alkenyl, Cycloalkylen-Oxy-Cycloalkenyl, Cycloalkylen-Oxy-Aryl, Cycloalkylen-Oxy-Heteroaryl, Heterocycloalkylen-Oxy-Alkyl, Heterocycloalkylen-Oxy-Cycloalkyl, Heterocycloalkylen-Oxy-Heterocyclen, Heterocycloalkylen-Oxy-Heterocycloalkyl, Heterocycloalkylen-Oxy-Alkenyl, Heterocycloalkylen-Oxy-Cycloalkenyl, Heterocycloalkylen-Oxy-Aryl, Heterocycloalkylen-Oxy-Heteroaryl, Arylen-Oxy-Alkyl, Arylen-Oxy-Cycloalkyl, Arylen-Oxy-Heterocyclen, Arylen-Oxy-Heterocycloalkyl, Arylen-Oxy-Alkenyl, Arylen-Oxy-Cycloalkenyl, Arylen-Oxy-Aryl oder Arylen-Oxy-Heteroaryl ist; oder
R₃ und R₄ zusammen mit dem Kohlenstoffatom, an das sie gebunden sind, einen 3-7-gliedrigen Ring bilden, der gegebenenfalls ein oder mehrere Heteroatome enthält, von denen jedes unabhängig voneinander S, 0 oder N ist; oder
R₁ und A zusammen mit dem Kohlenstoffatom, an das sie gebunden sind, einen 3-7-gliedrigen Ring bilden, der gegebenenfalls ein oder mehrere Heteroatome enthält, von denen jedes unabhängig voneinander S, 0 oder N ist; oder
R₂ und B zusammen mit dem Kohlenstoffatom, an das sie gebunden sind, einen 3-7-gliedrigen Ring bilden, der gegebenenfalls ein oder mehrere Heteroatome enthält, von denen jedes unabhängig voneinander S, 0 oder N ist; oder
jedes von A und B unabhängig voneinander Alkylen, Alkylen-Heteroalkylen, Alkenylen, Alkenylen-Heteroalkenylen, Alkylen-Heteroalkenylen, Alkynylen, Cycloalkylen, Alkylen-Cycloalkylen, Alkylen-Cycloalkylen, Alkenylen-Cycloalkylen, Alkenylen-Cycloalkylen-Alkenylen, Alkylen-Cycloalkylen-Alkenylen, Alken-Cycloalkylen-Alken, Alkynylen-Cycloalkylen-Cycloalkylen, Alkynylen-Cycloalkylen-Cycloalkylen, Heterocycloalkylen, Alkylen-Heterocycloalkylen, Alkylen-Heterocycloalkylen-Alkylen, Alkenylen-Heterocycloalkylen, Alkenylen-Heterocycloalkylen-Alkenylen, Alkylen-Heterocycloalkylen-Alken, Alkynylen-Heterocycloalkylen, Alkynylen-Heterocycloalkylen, Alkynylen-Heterocycloalkylen-Alkynylen, Cycloalkenylen, Alkylen-Cycloalkenylen, Alkylen-Cycloalkenylen-Alkylen, Alkenylen-Cycloalkenylen, Alkenylen-Cycloalkenylen, Alkenylen-Cycloalkenylen, Alkylen-Cycloalkenylen-Alkenen, Alkynylen-Cycloalkenylen, Alkynylen-Cycloalkenylen-Alkynylen, Heterocycloalkenylen, Alkylen-Heterocycloalkenylen, Alkylen-Heterocycloalkenylen-Alkylen, Alkenylen-Heterocycloalkenylen, Alkenylen-Heterocycloalkenylen, Alkylen-Heterocycloalkenylen-Alkenylen, Alkynylen-Heterocycloalkenylen, Alkynylen-Heterocycloalkenylen, Alkynylen-Heterocycloalkenylen, Arylen, Alkylen-Arylen, Alkylen-Arylen-Alkylen, Alkenylenarylen, Alkenylenarylenalkenylen, Alkylenarylenalkenylen, Alkynylenarylenarylen, Alkynylenarylenarylenalkynylen, Heteroarylen, Alkylenheteroarylen, Alkylenheteroarylenalkylen, Alkenylenheteroarylen, Alkenylenheteroarylenalkenylen, , Carbonyl oder Thiocarbonyl ist;
wobei optional m gleich 1 ist; jedes von R₁, R₂, R₃ und R₄ unabhängig Wasserstoff oder Alkyl ist; oder jedes A und B unabhängig Alkylen oder Alkenylen ist;
und wobei Alkyl, wenn es allein oder als Teil eines größeren Teils verwendet wird, mit einem oder mehreren Substituenten substituiert sein kann.

2. Isocyanatverbindung nach Anspruch 1, wobei das Isocyanat 2,4-Bis(isocyanatomethyl)-1,3-dioxolan ist.

3. Verfahren zur Herstellung einer Isocyanatverbindung nach einem der Ansprüche 1-2, umfassend den Schritt der Umwandlung einer Verbindung der Formel (I-A) in die Isocyanatverbindung, worin R, R₁, R₂, R₃, R₄, A, B und m die gleichen sind wie die in der Isocyanatverbindung

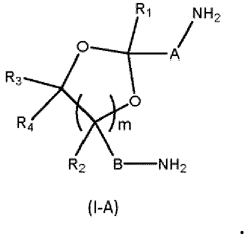

4. Verfahren nach Anspruch 3, wobei die Umwandlung der Verbindung der Formel (I-A) in die Isocyanatverbindung durch Umsetzung der Verbindung der Formel (I-A) mit Phosgen, Triphosgen oder Trichlormethylchlorformiat, gegebenenfalls in Gegenwart eines Katalysators, erfolgt;
wobei optional das Molverhältnis der Verbindung der Formel (I-A) zu Phosgen, Triphosgen oder Trichlormethylchlorformiat 1:2-100 beträgt, die Reaktionstemperatur im Bereich von -20-150 °C liegt; und der Katalysator, wenn vorhanden, ein Amin, ein Pyridinderivat oder N,N-Dimethylformamid umfasst.

5. Verfahren zur Herstellung einer Isocyanatverbindung nach einem der Ansprüche 1-2, umfassend den Schritt der Umwandlung einer Verbindung der Formel (I-B) in die Isocyanatverbindung, worin R, R₁, R₂, R₃, R₄, A, B und m die gleichen sind wie die in der Isocyanatverbindung, und jedes X unabhängig Hydroxy, Thiol oder Trimethylsiloxy ist.

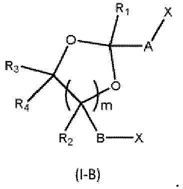

6. Verfahren nach Anspruch 5, wobei die Umwandlung der Verbindung der Formel (I-B) in die Isocyanatverbindung durch Umsetzung der Verbindung der Formel (I-B) mit Tetrabutylammoniumcyanat, gegebenenfalls in Gegenwart eines Katalysators, erfolgt;
wobei optional das Molverhältnis der Verbindung der Formel (I-B) zu Tetrabutylammoniumcyanat 1:2-100 beträgt, die Reaktionstemperatur im Bereich von -20-150 °C liegt und der Katalysator, wenn vorhanden, eine Triazinverbindung umfasst.

7. Abbaubares Polyurethan, wobei das Polyurethan hergestellt wird durch Polymerisieren einer Isocyanatverbindung nach einem der Ansprüche 1-2 mit einer wasserstoffabgebenden Verbindung, die einen zweiwertigen Alkohol, einen mehrwertigen Alkohol, Polyetherpolyol, Polyesterpolyol, binäres Mercaptan, mehrbasiges Mercaptan, Phenol, Carbonsäure, Harnstoff, Amid, Diamin oder Polyamin umfasst; und das Polyurethan eine spaltbare Vernetzungsstruktur der Formel (I-C) aufweist, worin R, R₁, R₂, R₃, R₄, A, B und m gleich sind wie die in der Isocyanatverbindung

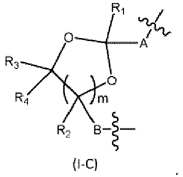

8. Abbaubares vernetztes Polymer, wobei das Polymer durch Polymerisieren einer Isocyanatverbindung nach einem der Ansprüche 1-2 mit einem Epoxidharz und einem abbaubaren Härtungsmittel hergestellt wird; wobei das Epoxidharz ein Glycidylether-Epoxidharz, ein Glycidylester-Epoxidharz, Glycidylepoxidharz, ein trifunktionales Epoxidharz, ein tetrafunktionelles Epoxidharz, ein Novolak-Epoxidharz, ein o-Kresol-Formaldehydharz, ein aliphatisches Epoxidharz, ein alicyclisches Epoxidharz oder ein stickstoffhaltiges Epoxidharz umfasst; und wobei der abbaubare Härter ein aliphatisches Acetal oder Ketalamin, ein aromatisches Acetal oder Ketalamin oder Salz davon, ein Acetal oder Ketalpolyamin, ein cyclisches Acetal oder Ketalamin, ein Acetal oder Ketalhydrazid oder Hydrazon umfasst; und wobei das vernetzte Polymer eine spaltbare Vernetzungsstruktur der Formel (I-C) aufweist, worin R, R₁, R₂, R₃, R₄, A, B und m gleich sind wie die in der Isocyanatverbindung

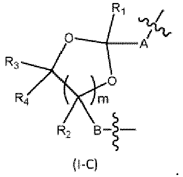

9. Verfahren zum Abbau eines Polyurethans nach Anspruch 7 oder eines vernetzten Polymers nach Anspruch 8, umfassend die folgenden Schritte:
(1) unter den Heiz- und Rührbedingungen wird das abbaubare Polyurethan oder das abbaubare vernetzte Polymer in ein gemischtes Säure- und Lösungsmittelsystem für den Abbau über 1-600 Stunden bei einer Temperatur im Bereich von 15-400 °C eingetaucht, wobei die Massenkonzentration der Säure im Lösungsmittel 0,1-99% beträgt;
(2) Verwenden einer Alkalilösung zum Einstellen des pH-Wertes der Abbaulösung auf über 6 bei einer Temperatur im Bereich von 0-200 °C, worin die Massenkonzentration der Alkalilösung 0,1-99% beträgt.

10. Verfahren nach Anspruch 9, worin die Säure Salzsäure, Bromwasserstoffsäure, Fluorwasserstoffsäure, Essigsäure, Trifluoressigsäure, Milchsäure, Ameisensäure, Propionsäure, Zitronensäure, Methansulfonsäure, p-Toluolsulfonsäure, Salpetersäure, Schwefelsäure, schweflige Säure, Phosphorsäure, Perchlorsäure, Benzoesäure, Salicylsäure oder Phthalsäure umfasst; das Lösungsmittelsystem Methanol, Ethanol, Ethylenglykol, Propanol, Isopropanol, Butanol, Isobutanol, t-Butanol, Pentanol, Hexanol, Heptanol, Octanol, Nonanol, Benzylalkohol, Phenethylalkohol, p-Hydroxymethylbenzol, m-Hydroxymethylbenzol, o-Hydroxybenzol, p-Hydroxyethylbenzol, m-Hydroxyethylbenzol, o-Hydroxyethylbenzol, Wasser, N,N-Dimethylformamid, N,N-Dimethylacetamid, N-Methylpyrrolidon, Dimethylsulfoxid, Tetrahydrofuran, Methyltetrahydrofuran, Glycerin oder Dioxan umfasst; das Alkali Lithiumhydroxid, Natriumhydroxid, Kaliumhydroxid, Calciumhydroxid, Natriumcarbonat, Natriumbikarbonat, Kaliumcarbonat, Kaliumcarbonat, Kaliumbikarbonat oder Ammoniak umfasst; das Lösungsmittel der Alkalilösung Methanol, Ethanol, Ethylenglykol, Propanol, Isopropanol, Butanol, Isobutanol, t-Butanol, Pentanol, Hexanol, Heptanol, Octanol, Nonanol, Benzylalkohol, Phenethylalkohol, p-Hydroxymethylbenzol, m-Hydroxymethylbenzol, o-Hydroxybenzol, p-Hydroxyethylbenzol, m-Hydroxyethylbenzol, o-Hydroxyethylbenzol, Wasser, N,N-Dimethylformamid, N,N-Dimethylacetamid, N-Methylpyrrolidon, Dimethylsulfoxid, Tetrahydrofuran, Methyltetrahydrofuran, Glycerin oder Dioxan umfasst;
und / oder wobei in Schritt (1) die Erwärmungstemperatur im Bereich von 80-150 °C liegt, die Erwärmungszeit im Bereich von 4-8 Stunden liegt und die Massenkonzentration von Säure im Lösungsmittel 0,5-20%; in Schritt (2) die Temperatur im Bereich von 5-50 °C liegt, der endgültige pH-Wert nach der Einstellung mit der Alkalilösung im Bereich von 6-12 liegt und die Massenkonzentration der Alkalilösung im Bereich von 5-30% liegt.

11. Recycelbares verstärktes Verbundmaterial, umfassend ein Polyurethan nach Anspruch 7 oder ein vernetztes Polymer nach Anspruch 8, ein Verstärkungsmaterial und ein Hilfsstoff, worin das Verstärkungsmaterial Kohlenstoff-Nanoröhrchen, Bornitrid-Nanoröhrchen, Ruß, Metall-Nanopartikel, Metalloxid-Nanopartikel, organische Nanopartikel, Eisenoxid, Glasfasern, Kohlenstofffasern, Naturfasern, synthetische Fasern und daraus hergestellte Gewebe umfasst; und das Hilfsmaterial einen Beschleuniger, ein Verdünnungsmittel, einen Weichmacher, ein Härtungsmittel, ein Verdickungsmittel, ein Kupplungsmittel, einen Entschäumer, ein Glättungsmittel, einen UV-Absorber, ein Antioxidans, einen Aufheller, ein Fluoreszenzmittel, ein Pigment oder einen Füllstoff umfasst.

12. Verfahren zum Recycling eines verstärkten Verbundmaterials nach Anspruch 11, umfassend die Schritte von:
(1) unter den Erwärmungs- und Rührbedingungen das verstärkte Verbundmaterial in eine Lösung einzutauchen, die eine Säure und ein Lösungsmittel umfasst, und dann das Gemisch für 1-600 Stunden auf eine Temperatur im Bereich von 15-400 °C zu erwärmen, um eine Zersetzungslösung zu erzeugen, worin die Massenkonzentration von Säure in der Lösung 0,1-99% beträgt;
(2) Verwenden einer Alkalilösung von 0-200°C, um den pH-Wert der Abbaulösung aus Schritt (1) auf größer als 6 einzustellen, um einen Niederschlag zu erhalten, worin die Massenkonzentration des Alkalis in der Alkalilösung 0,1-99% beträgt;
(3) Trennen, Waschen und Trocknen des in Schritt (2) erhaltenen Niederschlags;
wobei die Säure optional Salzsäure, Bromwasserstoffsäure, Flusssäure, Essigsäure, Trifluoressigsäure, Milchsäure, Ameisensäure, Propionsäure, Zitronensäure, Methansulfonsäure, p-Toluolsulfonsäure, Salpetersäure, Schwefelsäure, schweflige Säure, Phosphorsäure, Perchlorsäure, Benzoesäure, Salicylsäure oder Phthalsäure umfasst; das Lösungsmittel mindestens eines von Methanol, Ethanol, Ethylenglykol, Propanol, Isopropanol, Butanol, Isobutanol, t-Butanol, Pentanol, Hexanol, Heptanol, Octanol, Nonanol, Benzylalkohol, Phenethylalkohol, p-Hydroxymethylbenzol, m-Hydroxymethylbenzol, o-Hydroxybenzol, p-Hydroxyethylbenzol, m-Hydroxyethylbenzol, o-Hydroxyethylbenzol, Wasser, N,N-Dimethylformamid, N,N-Dimethylacetamid, N-Methylpyrrolidon, Dimethylsulfoxid, Tetrahydrofuran, Methyltetrahydrofuran, Glycerin oder Dioxan umfasst; das Alkali Lithiumhydroxid, Natriumhydroxid, Kaliumhydroxid, Calciumhydroxid, Natriumcarbonat, Natriumbikarbonat, Kaliumcarbonat, Kaliumbikarbonat, Kaliumbikarbonat oder Ammoniumhydroxid umfasst; und das Alkalilösungsmittel Methanol, Ethanol, Ethylenglykol, Propanol, Isopropanol, Butanol, Isobutanol, t-Butanol, Pentanol, Hexanol, Heptanol, Octanol, Nonanol, Benzylalkohol, Phenethylalkohol, p-Hydroxymethylbenzol, m-Hydroxymethylbenzol, o-Hydroxybenzol, p-Hydroxyethylbenzol, m-Hydroxyethylbenzol, o-Hydroxyethylbenzol, Wasser, N,N-Dimethylformamid, N,N-Dimethylacetamid, N-Methylpyrrolidon, Dimethylsulfoxid, Tetrahydrofuran, Methyltetrahydrofuran, Glycerin oder Dioxan umfasst;
wobei ferner optional in Schritt (1) die Massenkonzentration der Säure im Lösungsmittel im Bereich von 0,5-20% liegt, die Temperatur im Bereich von 80-200°C liegt und die Reaktionszeit 2-12 Stunden beträgt; und in Schritt (2) die Massenkonzentration der Alkalilösung im Bereich von 5-30% liegt, die Temperatur im Bereich von 5-60°C liegt.

## Revendications

1. Composé isocyanate de formule (I) : 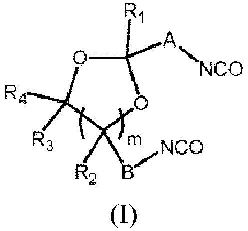 dans laquelle
m vaut 1, 2, 3, 4 ou 5 ;
chacun de R₁, R₂, R₃ et R₄ est indépendamment un hydrogène, alkyle, cycloalkyle, hétérocyclique, hétérocyclique, alcényle, cycloalcényle, alcynyle, aryle, hétéroaryle, alkylène-oxy-alkyle, alkylène-oxy-alkyle, alkylène-oxy-hétérocyclique, alkylène-oxy-hétérocycloalkyle, alkylène-oxy-alcényle, alkylène-oxy-cycloalcényle, alkylène-aryle, alkylène-oxy-hétéroaryle, cycloalkylène-oxy-alkyle, cycloalkylène-oxy-cycloalkyle, cycloalkylène-oxy-hétérocyclique, cycloalkylène-oxy-hétérocycloalkyle, cycloalkylène-oxy-alcényle, cycloalkylène-oxy-cycloalcényle, cycloalkylène-oxy-aryle, cycloalkylène-oxy-hétéroaryle, hétérocycloalkylène-oxy-alkyle, hétérocycloalkylène-oxy-cycloalkyle, hétérocycloalkylène-oxy-hétérocyclique, hétérocycloalkylène-oxy-hétérocycloalkyle, hétérocycloalkylène-oxy-alcényle, hétérocycloalkylène-oxy-cycloalcényle, hétérocycloalkylène-oxy-aryle, hétérocycloalkylène-oxy-hétéroaryle, arylène-oxy-alkyle, arylène-oxy-cycloalkyle, arylène-oxy-hétérocyclique, arylène-oxy-hétérocycloalkyle, arylène-oxy-alcényle, arylène-oxy-cycloalcényle, arylène-oxy-aryle, ou arylène-oxy-hétéroaryle ; ou
R₃ et R₄, conjointement avec l'atome de carbone auquel ils sont liés, forment un cycle à 3 à 7 chaînons contenant éventuellement un ou plusieurs hétéroatomes, chacun d'eux étant indépendamment S, O, ou N ; ou
R₁ et A, conjointement avec l'atome de carbone auquel ils sont liés, forment un cycle à 3 à 7 chaînons contenant éventuellement un ou plusieurs hétéroatomes, chacun d'eux étant indépendamment S, O, ou N ; ou
R₂ et B, conjointement avec l'atome de carbone auquel ils sont liés, forment un cycle à 3 à 7 chaînons contenant éventuellement un ou plusieurs hétéroatomes, chacun d'eux étant indépendamment S, O, ou N ; ou
chacun de A et B est indépendamment un alkylène, alkylène-hétéro-alkylène, alcénylène, alcénylène-hétéro-alcénylène, alkylène-hétéro-alcénylène, alcynylène, cycloalkylène, alkylène-cycloalkylène, alkylène-cycloalkylène-alkylène, alcénylène-cycloalkylène, alcénylène-cycloalkylène-alcénylène, alkylène-cycloalkylène-alcénylène, alcynylène-cycloalkylène, alcynylène-cycloalkylène-alcynylène, hétérocycloalkylène, alkylène-hétérocycloalkylène, alkylène-hétérocycloalkylène-alkylène, alcénylène-hétérocycloalkylène, alcénylène-hétérocycloalkylène-alcénylène, alkylène-hétérocycloalkylène-alcénylène, alcynylène-hétérocycloalkylène, alcynylène-hétérocycloalkylène-alcynylène, cycloalcénylène, alkylène-cycloalcénylène, alkylène-cycloalcénylène-alkylène, alcénylène-cycloalcénylène, alcénylène-cycloalcénylène-alcénylène, alkylène-cycloalcénylène-alcénylène, alcynylène-cycloalcénylène, alcynylène-cycloalcénylène-alcynylène, hétérocycloalcénylène, alkylène-hétérocycloalcénylène, alkylène-hétérocycloalcénylène-alkylène, alcénylène-hétérocycloalcénylène, alcénylène-hétérocycloalcénylène-alkylène, alkylène-hétérocycloalcénylène-alcénylène, alcynylène-hétérocycloalcénylène, alcynylène-hétérocycloalcénylène-alcynylène, arylène, alkylène-arylène, alkylène-arylène-alkylène, alcénylène-arylène, alcénylène-arylène-alcénylène, alkylène-arylène-alcénylène, alcynylène-arylène, alcynylène-arylène-alcynylène, hétéroarylène, alkylène-hétéroarylène, alkylène-hétéroarylène-alkylène, alcénylène-hétéroarylène, alcénylène-hétéroarylène-alcénylène, alkylène-hétéroarylène-alcénylène, alcynylène-hétéroarylène, alcynylène-hétéroarylène-alcynylène, carbonyle, ou thiocarbonyle ;
dans laquelle éventuellement m vaut 1 ; chacun de R₁, R₂, R₃ et R₄ est indépendamment un hydrogène ou alkyle ; ou chaque A et B est indépendamment un alkylène ou alcénylène ;
et dans laquelle l'alkyle, lorsqu'il est utilisé seul ou en tant que partie d'un fragment plus gros, peut être substitué par un ou plusieurs substituants.

2. Composé isocyanate selon la revendication 1, dans lequel l'isocyanate est le 2,4-bis(isocyanatométhyl)-1,3-dioxolane.

3. Procédé pour préparer un composé isocyanate de l'une quelconque des revendications 1 et 2, comprenant l'étape de conversion d'un composé de formule (I-A) en le composé isocyanate, où R, R₁, R₂, R₃, R₄, A, B et m sont les mêmes que ceux dans le composé isocyanate.

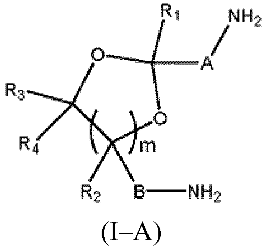

4. Procédé selon la revendication 3, dans lequel la conversion du composé de formule (I-A) en le composé isocyanate a lieu par réaction du composé de formule (I-A) avec du phosgène, du triphosgène, ou du chloroformiate de trichlorométhyle, éventuellement en présence d'un catalyseur ; dans lequel éventuellement le rapport molaire du composé de formule (I-A) au phosgène, triphosgène ou chloroformiate de trichlorométhyle est de 1/2 à 100, la température réactionnelle est située dans la plage allant de -20 à 150°C ; et le catalyseur, lorsqu'il est présent, comprend une amine, un dérivé de pyridine, ou du N,N-diméthylformamide.

5. Procédé pour préparer un composé isocyanate de l'une quelconque des revendications 1 et 2, comprenant l'étape de conversion d'un composé de formule (I-B) en le composé isocyanate, où R, R₁, R₂, R₃, R₄, A, B et m sont les mêmes que ceux dans le composé isocyanate, et chaque X est indépendamment hydroxyle, thiol ou triméthylsiloxy.

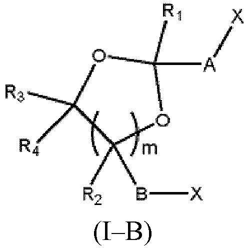

6. Procédé selon la revendication 5, dans lequel la conversion du composé de formule (I-B) en le composé isocyanate a lieu par réaction du composé de formule (I-B) avec du cyanate de tétrabutylammonium, éventuellement en présence d'un catalyseur ;
dans lequel éventuellement le rapport molaire du composé de formule (I-B) au cyanate de tétrabutylammonium est de 1/2 à 100, la température réactionnelle est située dans la plage allant de -20 à 150°C ; et le catalyseur, lorsqu'il est présent, comprend un composé triazine.

7. Polyuréthane dégradable, lequel polyuréthane est obtenu par polymérisation d'un composé isocyanate de l'une quelconque des revendications 1 et 2 avec un composé donneur d'hydrogène qui comprend un alcool divalent, un alcool polyvalent, un polyéther-polyol, un polyester-polyol, un mercaptan binaire, un mercaptan polybasique, un phénol, un acide carboxylique, une urée, un amide, une diamine, ou une polyamine ; et le polyuréthane a une structure de réticulation pouvant être coupée de formule (I-C), dans laquelle R, R₁, R₂, R₃, R₄, A, B et m sont les mêmes que ceux dans le composé isocyanate.

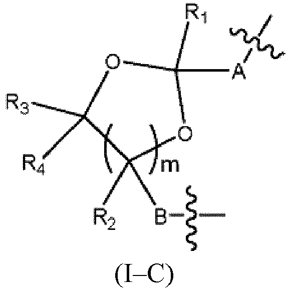

8. Polymère réticulé dégradable, lequel polymère est obtenu par polymérisation d'un composé isocyanate de l'une quelconque des revendications 1 et 2 avec une résine époxy et un agent durcisseur dégradable ; dans lequel la résine époxy comprend une résine époxy de glycidyléther, une résine époxy de glycidylester, une résine époxy de glycidylépoxyamine, une résine époxy trifonctionnelle, une résine époxy tétrafonctionnelle, une résine époxy novolaque, une résine époxy d'o-crésolformaldéhyde, une résine époxy aliphatique, une résine époxy alicyclique, ou une résine époxy azotée ; et l'agent durcisseur dégradable comprend une amine aliphatique d'acétal ou de cétal, une amine aromatique d'acétal ou de cétal ou un sel de celle-ci, une polyamine d'acétal ou de cétal, une amine d'acétal ou de cétal cyclique, un hydrazide d'acétal ou de cétal, ou de l'hydrazone ; et le polymère réticulé a une structure de réticulation pouvant être coupée de formule (I-C), dans laquelle R, R₁, R₂, R₃, R₄, A, B et m sont les mêmes que ceux dans le composé isocyanate.

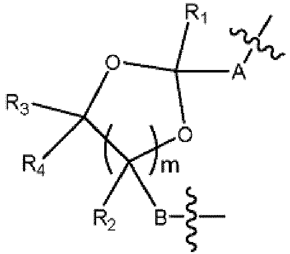

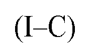

9. Procédé pour dégrader un polyuréthane de la revendication 7 ou un polymère réticulé de la revendication 8, comprenant les étapes suivantes :
(1) dans des conditions de chauffage et d'agitation, le polyuréthane dégradable ou le polymère réticulé dégradable est immergé dans un système mixte d'acide et de solvant pour la dégradation pendant 1 à 600 heures à une température située dans la plage allant de 15 à 400°C, la concentration en masse d'acide dans le solvant étant de 0,1 à 99 % ;
(2) utilisation d'une solution alcaline pour ajuster le pH de la solution de dégradation au-delà de 6 à une température située dans la plage allant de 0 à 200°C, la concentration en masse de la solution alcaline étant de 0,1 à 99 %.

10. Procédé selon la revendication 9, dans lequel l'acide comprend de l'acide chlorhydrique, de l'acide bromhydrique, de l'acide fluorhydrique, de l'acide acétique, de l'acide trifluoroacétique, de l'acide lactique, de l'acide formique, de l'acide propionique, de l'acide citrique, de l'acide méthanesulfonique, de l'acide p-toluènesulfonique, de l'acide nitrique, de l'acide sulfurique, de l'acide sulfureux, de l'acide phosphorique, de l'acide perchlorique, de l'acide benzoïque, de l'acide salicylique, ou de l'acide phtalique ; le système solvant comprend du méthanol, de l'éthanol, de l'éthylèneglycol, du propanol, de l'isopropanol, du butanol, de l'isobutanol, du t-butanol, du pentanol, de l'hexanol, de l'heptanol, de l'octanol, du nonanol, de l'alcool benzylique, de l'alcool phénéthylique, du p-hydroxyméthylbenzène, du m-hydroxyméthylbenzène, de l'o-hydroxybenzène, du p-hydroxyéthylbenzène, du m-hydroxyéthylbenzène, de l'o-hydroxyéthylbenzène, de l'eau, du N,N-diméthylformamide, du N,N-diméthylacétamide, de la N-méthylpyrrolidone, du diméthylsulfoxyde, du tétrahydrofurane, du méthyltétrahydrofurane, du glycérol, ou du dioxane ; l'alcali comprend de l'hydroxyde de lithium, de l'hydroxyde de sodium, de l'hydroxyde de potassium, de l'hydroxyde de calcium, du carbonate de sodium, du bicarbonate de sodium, du carbonate de potassium, du bicarbonate de potassium, ou de l'ammoniac ; le solvant de la solution alcaline comprend du méthanol, de l'éthanol, de l'éthylèneglycol, du propanol, de l'isopropanol, du butanol, de l'isobutanol, du t-butanol, du pentanol, de l'hexanol, de l'heptanol, de l'octanol, du nonanol, de l'alcool benzylique, de l'alcool phénéthylique, du p-hydroxyméthylbenzène, du m-hydroxyméthylbenzène, de l'o-hydroxybenzène, du p-hydroxyéthylbenzène, du m-hydroxyéthylbenzène, de l'o-hydroxyéthylbenzène, de l'eau, du N,N-diméthylformamide, du N,N-diméthylacétamide, de la N-méthylpyrrolidone, du diméthylsulfoxyde, du tétrahydrofurane, du méthyltétrahydrofurane, du glycérol, ou du dioxane ;
et/ou dans lequel, dans l'étape (1), la température de chauffage est située dans la plage allant de 80 à 150°C, le temps de chauffage est situé dans la plage allant de 4 à 8 heures, et la concentration en masse de l'acide dans le solvant est de 0,5 à 20 % ; dans l'étape (2), la température est située dans la plage allant de 5 à 50°C, la valeur de pH finale après ajustement avec la solution alcaline est située dans la plage allant de 6 à 12, et la concentration en masse de la solution alcaline est située dans la plage allant de 5 à 30 %.

11. Matériau composite renforcé recyclable comprenant un polyuréthane de la revendication 7 ou un polymère réticulé de la revendication 8, un matériau de renforcement, et un matériau auxiliaire, dans lequel le matériau de renforcement comprend des nanotubes de carbone, des nanotubes de nitrure de bore, du noir de carbone, des nanoparticules métalliques, des nanoparticules d'oxyde métallique, des nanoparticules organiques, de l'oxyde de fer, des fibres de verre, des fibres de carbone, des fibres naturelles, des fibres synthétiques et une étoffe produite à partir de celles-ci ; et le matériau auxiliaire comprend un accélérateur, un diluant, un plastifiant, un agent de ténacité, un agent épaississant, un agent de couplage, un anti-moussant, un agent de planéité, un agent absorbant les ultraviolets, un antioxydant, un azurant, un agent fluorescent, un pigment, ou une charge.

12. Procédé pour recycler un matériau composite renforcé de la revendication 11, comprenant les étapes suivantes :
(1) dans des conditions de chauffage et d'agitation, l'immersion du matériau composite renforcé dans une solution comprenant un acide et un solvant et ensuite le chauffage du mélange à une température située dans la plage allant de 15 à 400°C pendant 1 à 600 heures pour donner une solution de dégradation, la concentration en masse de l'acide dans la solution étant de 0,1 à 99 % ;
(2) l'utilisation d'une solution alcaline à 0-200°C pour ajuster le pH de la solution de dégradation obtenue dans l'étape (1) de façon qu'il soit supérieur à 6 afin que soit obtenu un précipité, la concentration en masse de l'alcali dans la solution alcaline étant de 0,1 à 99 % ;
(3) la séparation, le lavage et le séchage du précipité obtenu dans l'étape (2) ;
dans lequel éventuellement l'acide comprend de l'acide chlorhydrique, de l'acide bromhydrique, de l'acide fluorhydrique, de l'acide acétique, de l'acide trifluoroacétique, de l'acide lactique, de l'acide formique, de l'acide propionique, de l'acide citrique, de l'acide méthanesulfonique, de l'acide p-toluènesulfonique, de l'acide nitrique, de l'acide sulfurique, de l'acide sulfureux, de l'acide phosphorique, de l'acide perchlorique, de l'acide benzoïque, de l'acide salicylique, ou de l'acide phtalique ; le solvant comprend au moins l'un parmi le méthanol, l'éthanol, l'éthylèneglycol, le propanol, l'isopropanol, le butanol, l'isobutanol, le t-butanol, le pentanol, l'hexanol, l'heptanol, l'octanol, le nonanol, l'alcool benzylique, l'alcool phénéthylique, le p-hydroxyméthylbenzène, le m-hydroxyméthylbenzène, l'o-hydroxybenzène, le p-hydroxyéthylbenzène, le m-hydroxyéthylbenzène, l'o-hydroxyéthylbenzène, l'eau, le N,N-diméthylformamide, le N,N-diméthylacétamide, la N-méthylpyrrolidone, le diméthylsulfoxyde, le tétrahydrofurane, le méthyltétrahydrofurane, le glycérol, ou le dioxane ; l'alcali comprend de l'hydroxyde de lithium, de l'hydroxyde de sodium, de l'hydroxyde de potassium, de l'hydroxyde de calcium, du carbonate de sodium, du bicarbonate de sodium, du carbonate de potassium, du bicarbonate de potassium, ou de l'hydroxyde d'ammonium ; le solvant de la solution alcaline comprend du méthanol, de l'éthanol, de l'éthylèneglycol, du propanol, de l'isopropanol, du butanol, de l'isobutanol, du t-butanol, du pentanol, de l'hexanol, de l'heptanol, de l'octanol, du nonanol, de l'alcool benzylique, de l'alcool phénéthylique, du p-hydroxyméthylbenzène, du m-hydroxyméthylbenzène, de l'o-hydroxybenzène, du p-hydroxyéthylbenzène, du m-hydroxyéthylbenzène, de l'o-hydroxyéthylbenzène, de l'eau, du N,N-diméthylformamide, du N,N-diméthylacétamide, de la N-méthylpyrrolidone, du diméthylsulfoxyde, du tétrahydrofurane, du méthyltétrahydrofurane, du glycérol, ou du dioxane ;
dans lequel en outre éventuellement, dans l'étape (1), la concentration en masse de l'acide dans le solvant est située dans la plage allant de 0,5 à 20 %, la température est située dans la plage allant de 80 à 200°C, et le temps de réaction est de 2 à 12 heures ; et dans l'étape (2), la concentration en masse de la solution alcaline est située dans la plage allant de 5 à 30 %, la température est située dans la plage allant de 5 à 60°C.
